# EUROPEAN PATENT APPLICATION

(11) **EP 2 535 717 A1**
(43) Date of publication of application: **19.12.2012**
(21) Application number: 11169674.6
(22) Date of filing: 13.06.2011
(51) Int. Cl.: G01N 33/68, C12Q 1/68, C12Q 1/52

(54) **Method for determining the mortality risk**

(71) Applicant: Universitätsklinikum Hamburg-Eppendorf, 20246 Hamburg (DE); Friedrich-Alexander-Universität Erlangen-Nürnberg, 91054 Erlangen (DE); Technische Universität Dresden, 01307 Dresden (DE)
(72) Inventor: Böger, Rainer, 22459 Hamburg (DE); Schwedhelm, Edzard, 20249 Hamburg (DE); Lüneburg, Nicole, 22089 Hamburg (DE); Maas, Renke, 91056 Erlangen (DE); Rodionov, Roman, 01309 Dresden (DE)
(74) Representative: UEXKÜLL & STOLBERG

(57) **Abstract**

The present invention generally relates to methods for determining the mortality risk in a subject. In one aspect, the invention relates to a method for determining/predicting the all-cause mortality risk of a test subject based on the detection of pathologically decreased expression levels of the gene encoding the alanine-glyoxylate-aminotransferase 2 (AGXT2) and/or pathologically decreased activity levels of the AGXT2 enzyme. In a further aspect, the invention relates to a method for determining/predicting the all-cause mortality risk of a test subject based on the detection of genetic alterations which are associated with such pathologically decreased expression levels and/or activity levels. Finally, the invention also relates to compounds which are effective in increasing the expression of the AGXT2 gene and/or the activity of the AGXT2 enzyme for use in a method of decreasing the mortality risk of a subject.

SEQUENCE LISTING

<110>  Universitätsklinikum Hamburg-Eppendorf
       Friedrich-Alexander-Universität Erlangen-Nürnberg
       Technische Universität Dresden

<120>  METHODS FOR DETERMINING THE MORTALITY RISK

<130>  P 84721

<160>  18

<170>  PatentIn version 3.3

<210>  1
<211>  52
<212>  DNA
<213>  Homo sapiens


<220>
<221>  variation
<222>  (27)..(27)
<223>  SNP: G replaced by C

<400>  1
cctccatggg aggcagtgtc tcaaacgtca atatcagtga gggacttcaa aa          52


<210>  2
<211>  52
<212>  DNA
<213>  Homo sapiens


<220>
<221>  variation
<222>  (27)..(27)
<223>  SNP: C replaced by T

<400>  2
ttcatgcatt ggagggtgga agaagacggt gcttgtatgc cacaggcggc cg          52


<210>  3
<211>  52
<212>  DNA
<213>  Homo sapiens


<220>
<221>  variation
<222>  (27)..(27)
<223>  SNP: T replaced by C

<400>  3
gttttaaaaa ataagtcatt taataatgtt tggcaatttc cttttaataa ta          52


<210>  4
<211>  52
<212>  DNA
<213>  Homo sapiens


<220>
<221>  variation
<222>  (27)..(27)
<223>  SNP: T replaced by C

<400>  4
tcccggaaaa gaaatccagg tatctgtttc cttcagcatc aaagagccac tc          52

<210>  5
<211>  52
<212>  DNA
<213>  Homo sapiens

<220>
<221>  variation
<222>  (27)..(27)
<223>  SNP: G replaced by A

<400>  5
gtgtgtgttt ggatgagcaa gcacaagcag gcacagacct aggtagagta gg          52

<210>  6
<211>  52
<212>  DNA
<213>  Homo sapiens

<220>
<221>  variation
<222>  (27)..(27)
<223>  SNP: G replaced by A

<400>  6
tttttgcaag gccaggtgat gaggatggca ttcagtctag gtagtgtggt tt          52

<210>  7
<211>  52
<212>  DNA
<213>  Homo sapiens

<220>
<221>  variation
<222>  (27)..(27)
<223>  SNP: T replaced by G

<400>  7
aggaggtagg aggagagtgg aaattatgcc agaaagaaaa tatgtttttt aa          52

<210>  8
<211>  52
<212>  DNA
<213>  Homo sapiens

<220>
<221>  variation
<222>  (27)..(27)
<223>  SNP: A replaced by T

<400>  8
tgaagatcag tgatccttaa ttacctaaga aggctcagta catttttact tt          52

<210>  9
<211>  52
<212>  DNA
<213>  Homo sapiens

<220>
<221>  variation
<222>  (27)..(27)
<223>  SNP: T replaced by C

<400>  9
ctaggtcgct gaacacatcc acatgttggg agagtagcac accccagcat gg          52


<210>  10
<211>  52
<212>  DNA
<213>  Homo sapiens


<220>
<221>  variation
<222>  (27)..(27)
<223>  SNP: A replaced by G

<400>  10
ctctggacgc ttccagacct tgccctatac acctcttctt ctggctgttc at          52


<210>  11
<211>  52
<212>  DNA
<213>  Homo sapiens


<220>
<221>  variation
<222>  (27)..(27)
<223>  SNP: T replaced by C

<400>  11
atctactttt attcctgttc ccacttttgt tcaggtatcc atcctaagcg ga          52


<210>  12
<211>  49993
<212>  DNA
<213>  Homo sapiens

<400>  12
ctctttttct cccccttgaa ggcactgtta ctagaaatag agagctctgg ggcttgcctg      60

aagccatggg cagtcaccac tccaggattc cccttttagc gcctcctccc catcttaatc     120

tcaaagtcca gcaggatagt gagcagttag agcccggcct ccaatctgct tccatggggg     180

ttggctttct gagtgggaga aatgactcta atctggagac atttgctgag acccttgtgc     240

ctggtcactt ccgctcccag gatccttgag atgcatcctt tcctgagccg taagtggaaa     300

ccagggggac gtgggtcagt agaggatcag cgagagggta agacttgagc tccgaaggct     360

gctgcctggg attctggaat gttagacctg ggtgaggtct tagagatgag gcatgttttt     420

gacacacagg ttctcaccct gagtcaatgc cccattttca tagccaatat tttgtaatgc     480

tcctttttgt tttgaaatga aattcgtgga taatgaaacc cgactataca caatttcaac     540

atgacatgct atctcagctt caatattttt aaaaaccaaa ggaaagcaat caaaatagag     600

tgtgacttca atttacagat gcccaaggat gatttctatt ttttcttttt tgagacaggg     660

tctccctctg tcacctaggt tggagtacag tggtgtgatc atggttcact gcagccttga 720
cctccaaggc tcaaataatt ctcccacctc agcctcctga gtagctggaa ctataggcac 780
atgccaccat gcctagctat ttttcttttt taacttttgt agacatgggg ttttactatg 840
ttgctcaagt tggtctcgaa ctcccgggct caagcaatcc tctcacttca gtctttcaaa 900
gtgctcggat tgcaggccta agccattgca ccaggctagg atgactattt taaaagatgt 960
aaagcatctg catgaatttt catcatgcct ctaggaagtg agaccacgct ctcaattgcc 1020
aacaagtgct gtgattcacc cagagtcatc caagccaggg cttgacccca ggccctcccc 1080
atctactcag actctcctct ctgcagcaag aacagaaaat atgaggagat gtggggtaac 1140
cagacattgg tggctttatg agtaatatcc tatatgcttg tgattttcac atggatcttg 1200
tttcacgaga gcctcacctc ctcctcagca aattccctca tgcaaacatg agattaagca 1260
actgtctgta catttatgtg cctgtgcttc acgggccgta aacaggtaca ggggaccatt 1320
ttacattccc cattgtgacc aactttgcct tcattttctc ctcatatttc cctttcaccc 1380
tccttccttc ctctcctctt cgctttctta cccctgaaaa ttgcagtttt ttgaaacttg 1440
acacatgcat gatgctttaa atgcattatc tccttttatc ttcaaccaaa cttgttagtg 1500
agtactatta tctcattcca caaatgagga gacaagagaa taaggaatat ctaagaaatg 1560
tggtcaaagg cagaaacatg tatgtgataa cttgggattc aaagccaggt ctatctgact 1620
ccgaactcga agagattcta ccaggccctg tctatcctcc cctagtcttc caatcctgtt 1680
tggactgctc ttttcccttg gtcatgagtt tcttttgcca gaacatcacc taactccaac 1740
attttctcag aagcaggtaa aattataacc agtcttatgc tcagtgagga ggaaattatg 1800
ctgaaagtac cagctgttga cctagcaaga gaaatgccct agactgcccc ctccccttcc 1860
ctaccccaac ctctgaacat tgtggagttg cacagtggag cagcaggtaa cctagagagg 1920
gcaggtgagg cctagtacct agagatcttg agcaactcgt tggcgacgcc ttaccatagg 1980
cccagcaagg acactacagg gaagcagtct tggaaaacca caccactggc cgggtgcggt 2040
ggctcacacc tctaatccca gcactttgtg aggctgaggc gggcggatca cgaggtcagg 2100
agatcgagac catcctggct aacacgggga aaccctgtct gtactaaaag tacaaaaaat 2160
tagtcgggca tggtggcgaa cgcctgtagt tccagctact caggaggctg aggcaggaga 2220
atggcttgaa cccgggagtc ggagcttgca gtgagcggag attgcgccac tgcactccag 2280
cctgggcgac agagtgagac tccttctcaa aaaaaaaaaa aaaaaaaaag aaaaaagaaa 2340
aagaaaaaaa ccacactacc tagactgaat gctatcctca tcacctggcc ttgcaaaaac 2400
ctttgggact tttatgtcta ctttcttacc tactctacct aggtctgtgc ctgtttgtgc 2460
ttgctcatcc aaacacacac tcactagcat acatttattg agcacctgcc atgtgcaata 2520
tatacattca ggcaaaatat ttgagcatct attatgcacc agtgctgcag acttagccac 2580
aataagcaag aaaaagctcc tgccctctat tccaggaagg agatagacat aaatgaataa 2640
gtaaatatgt aaaaatatat atgtgtgtga atatgtatgt atttatgtat gtatccaata 2700
gagacacatg ccatgaataa aaccaaatct ggaaagggaa tagaaaaata taatgggggt 2760

gaggggaggg gctgtgatag ggaggtcacc acgttttaag tgttgtttta tatgcaaaaa 2820
taaataagtc acaattcttg tatagtatct acatagttac tgtacaatat tcatatgcat 2880
aaaatgttca gctttgttta cactttgata tgcacaatca ctttgggcta actgtgtata 2940
caaagttatg gaagcaattt gttttcagta caaaagtgtg gagaaatatc tcagtggggc 3000
attctttcct acaattagct aatgactgaa atatataaca tttgcaggtg gctggggtat 3060
gtgctcccat gtaccaattt cctaatatat gcctgctctc ccacaaagga aggagggcac 3120
aaaaagtcat ctatttccag ctgagatgtt tctccattcc tgccaagcct attttaagaa 3180
aacatttaaa atttgcccta aaggggggcc tcccatctac acccctccag gcagccttgc 3240
cacagtcaga actgatttaa cacatgccaa gggccaggaa gggtgatcac agagaatgcc 3300
tactcccgaa cgtgcggaag aagacccacc cacctcagaa tcaggaatcg aggagtgctc 3360
ttgcttgtgg acagccacaa ctgcccctgt gtggcttcag caaaagggca tatactggaa 3420
ggttatgaag gaatggaggg aaggagggag ggcagctgaa ataatcaaca ggaggctgag 3480
gacgtggaca ggaccccaca cgcaaagaag ccaaggaagg ccaggcagga gtagagtaac 3540
ttctggtagg aaccgcctct tagcagcttt gcttagcgga tgcaggcacc tggcattcac 3600
tgccatcatc cttgcaccca gagcacgcat gggacagagg ggacctccac agagaaggct 3660
gtgagagatc atgtccggag cggatgatac cgaacagcaa tggtgagggt cctcagcagc 3720
gtgctgcaga ctgcggcccc atgtgtgaag agaaaacgac cactctggag tctccaagct 3780
ggcagctcca gcacagagaa gcagaggaga aaaatttctc taccgaccag acactgtccc 3840
tcaggcgtcc aaaccccaac acagcttcag caggcctcca tgggaggcag tgtctcaaac 3900
gtcaatatca gtgagggact tcaaaactgc caaggcctcg aattccatct gtcactgatg 3960
tcctctaagg tggatggagg atgaaatctt cctttcctaa ctcgttggca atcccaagta 4020
tcaaatgaga ttgcaaatgt gacaaggttt ttttcaagtt acctaagatg ctatacagaa 4080
atgagataca attggcattt tgggccaggc ttagtggttc acgcctgtaa tcacaacact 4140
tccagaggcc aaggcagaca gatggcttga tctcacaagt ttgagatcag cctgggcaat 4200
ctggagaaac ccgatctcta ctaaaaatac aaaacactag ccgggtgtgg tggcatgccc 4260
ctatggtccc agctactaag gaggctgaga tagaaggatc gcttgagcct gagaggtaga 4320
ggttgcaatg agcagagatt ccaccactgc actccatgta agaccccatg tctattaaaa 4380
gtaggaaaag aaaaatagct gggcatggtg gctggtgcct atggttccag ctactcaaga 4440
ggctgaggtg ggagaattgt ttgagcctgg gaggccgagg ctacagtaag ccatgctcac 4500
accactgtac tccagcctgg gtgacagaag gagatcctgt ctcaaaaaag aaaaaaagaa 4560
agaaatcatc cctgggcatc tgtaaattaa agccataatt tattttgaaa gcttttcttt 4620
gatttttaaa attattgcag ctgagatagc atgtcattat tttcacacat tatatgagca 4680
ttgggcaaca gagccagacc tgtctcagta ataataataa ttagcatttt aataaatcac 4740
actgtgtcca ctgcatattt tcaaatattt tagaatcttt ttttcatttt aaagggtgag 4800

gaagccctta ataaatactc cctctgggct gaaaactggt tgtgaatact gaaaaactcg 4860
ttatcattac aaagagaatt ttccctagag tttccaaatg ccaaaaaaat ttgatttttc 4920
tcatttactt tgcccaacag tttttgtcat tcgatattat gattttgggg aaagaaaata 4980
aagttgcagt gaactgtctg ctatagaact ctgtgagaat taggcagctt ggaaatgaaa 5040
gctcactagt tacccacttg gcatttatta tgcaaaagcg atgctgttct ccaaattgaa 5100
ttccatcttt caatgtctca agttgctcat gtgaattgaa gtgaaaactg gtgaattgga 5160
tgaaaatcat tagctgtcgt ctggttagcc taaaaaggca ctatccgatt agttaaaaca 5220
ataagacaaa acaaaacaaa tacctattca gaaatagacc ttggatatag ttttattaat 5280
cctaatctca ttcattcctt cccctccacc tccatctctt acacacacac acacacacac 5340
acacacacac acacacaggt atgcatatgc atatatacac atacacactt tccaggagcc 5400
cccaaaaaag ctttatgaac cctcaactct tacatcatcc tcaaaacaca ggctattttg 5460
aataaaaatt gcatgcaatt atctagatag attttcattt tttttctggc aaataataaa 5520
aaccaaagta gttttgtttc tttctgtcca tcaaatatca acattattac tattattttt 5580
gagttttagt tatttattaa gaggagtgat atgacagtca atagatttgg aggtattatc 5640
caggtttatt tcacttactg actcctatta ttctttctcc tacttgctca ttactgtctc 5700
tatttcttcc ttcctctttc ttctttcttt cctccttctt tcctttcttc ctcttctttc 5760
ttccttgcag aatttcacgt atttcatttg aagccctcac tccaccatca tgtgcttatg 5820
acttttgtga ataaatgaat gaataaaact gccttgactg tttaaaattc aagcttttct 5880
ttgtcaagta tttctctcca actcccttcc ccctccagcc tactttcata aatcctataa 5940
ccttggagtc aagacagctt acaacctcct caatgggcaa ctccaatttt gcagatagaa 6000
aaatgagact gaagaaacga aaggattttt gaattcttag agacactcag aatttttttt 6060
ccatctcaga agcacaacga atgcagagag acacaatgaa aagcttttta aaaggtgaaa 6120
ttctcagaaa actaaagcta tcaatttttt tggtcctttc tatgcatgga taaccaaaag 6180
tcacttgctt ttcaagtaaa agcaaattaa tgacattagt gattatatat ctctttgaaa 6240
tatatatctc tttgaatata tctctttgaa agaagatata ttcagaactg ttactttcca 6300
tcttcattta aaaacacaat tgttcattat gtttctcagg actgtgactt catagccaca 6360
caaagcacta acacttcttt catgcattca aaccttcatg ctgagaaaga gacataattt 6420
ccttagctct ctccacctat aaaattatct taatgttgac cttcagtctt caaaattagg 6480
agcatctttc ctaggagtac ttataaggtc tctagacaac tcttccagct ctgacgttct 6540
gattccatta ttccatccca ctgctgcagg ggggccaaag tctgtgctgg cagggaaggg 6600
tggaaatggt tgaggaatga gcaaaacaga ctactgggct aggagtcaga ggagttttgt 6660
tcccgttctt cacctaacta gcttgataac cttaaacaga ttaatttacc actttacgct 6720
ggtttcctta tctataaagt atgcattttg acacacacac acacacaaat gtaaggtccc 6780
tcctagctcc aaatatttaa tgaatctggt tcagcttatc cctgtttcat cacagtctta 6840
tgttggcagc cttttttttt tttttttttt ggcgggggga ggttttgtct tctgggtcta 6900

tttgcatgta tttattagtc tagtatattg tctaacacct gcctctcctc attccctaca 6960
agaatagtca acagtcatta atgccgtgtg tattgagagc taaccaggta ccaggcactg 7020
gctacacact agaattcagg cactaggcag cttgaagcct aaaaagcacc tctattaaca 7080
ggggatggac tccaacaacg cattgcacta gcagaagttt tgcctactga tgagagaatc 7140
gacacggtga gggtaacatg gagagtttgc ctaaactgag cctcccaagt ttgtgaggga 7200
ctgccgtaga ttttttttcc agactatatt tgagaagaac tatttttttc ctccagaaaa 7260
ctcattattg agatactcag attttaaatc caaactaaaa gctgattagc atgatttttt 7320
aactaagaaa taaaattatc taagcagttt tatttggata actataggtg actttcacaa 7380
acagacctat gtcatgctta gttgaggatt ctaactcact tcactttcag taggtacttc 7440
ccggacatca gtaaccaagc tcagtcttca tacaaagccc agaatgcctc catgtgactt 7500
catgcctgaa agataccagg tgagattcag ggctttaaga aaactcaaag aggtagtttc 7560
tctggggagg tctcaaatga ttgtttcctt aagaatgaca caaaaatagc cctgcttgcc 7620
actaaactac ctcattccag atattgtacc tctaagtcat tggaagcatc tgaaaccctc 7680
ttaatccctt aatgctagtg tataaattgg tgtccatgtg tgagagatag agacacagac 7740
agaggcaaag aaagtacaag aagcacacta cacatgataa ttaatatatg tactgcaggg 7800
agatatacac acatcacaaa tgtgatggca cacataggct ttaaagtaaa tgttgctcct 7860
gttttaactg actcatgtgg ccatctgacc ttcctgggta ctccagcaaa aattatagtt 7920
atatttttgt tctcaggcta ataaaaattc agctggcatt ttctctctta ctttctctct 7980
ctttctttct ttctctctct ttctttctcc ctctctctct ctctctctct gttatgcatg 8040
cacacgtgca tgcacacaca cacggcaact cctttattcg acatgatact tacaggttag 8100
aaattgtaga atcaataggt atttatgagg gatccacaaa gaagagaaat acaggagaga 8160
tattttataa ataaagatac aaacacgact agcacttcat tgtattttaa ttttttaaaa 8220
cactaccaca acaaaaatga cttattagtt cagactaatt gcttattagt tcagacattt 8280
acttccgaag tctatttttt tcatagttaa aatcaactca gagccaaggc ttttcattgc 8340
taggtagaaa ttccagccct tttctgaaaa gagatcgaaa ggctacaaat aataattcac 8400
ctccttaaga gaaaaggctt taatttcttg cagggacgat attgggtaag tacacgcact 8460
tctgagcctc tacaggccag cagccctctt ctctgtccac tagagagcct gggctctgtt 8520
actcatagat tgactgctat tgatcttgta agaagtgtgt gggtttaaat cttgtttatc 8580
tacagtccct tggctacaac cgtgtcctgg aaatccacaa ggaacatctt tctcctgtgg 8640
tgacggcata tttccagaaa cccctgctgc tccaccaggg gcacatggag tggctctttg 8700
atgctgaagg aagcagatac ctggatttct tttccgggat tgttactgtc agtgttggcc 8760
attgccaccc gtgagtatcc ttaaaatctg gagattttta ttccaaaaga atgcatggaa 8820
aacaattatg ttagtgactc tgaactcttg atttgcttat cttggtttaa aaatatctat 8880
gacagattgt ggctgacata ctccctgcta tttcctgagt acaaaatctt gacaccctta 8940

cttttccttt cttgacaatg caagattttg ttattctcat ggcacatgta gtgtggcatg 9000
taccatgtga ctggagtctt gctgtgtgtg tttctcatta gcaacagctt tccaaagaga 9060
ggggtgcatt cagggaaagg aagagaaagg caggcggctc tcagagaaac acatgacagg 9120
cttgtcaggc gagggatgat gagagatgag aaataacttc tagcctagag aatcttcttc 9180
atggcctctg agagatggaa gaggaaaaga agaatgataa gagacagatg caatcctagc 9240
tggaaattta cctggaaatg tatggatcaa ttaatctaga gattgtctgc agtgttgacg 9300
gtatactcgc ccacacagat acccttggag cgttcttggt ttccatgcag tgctctcatt 9360
tcatgtctgt tctctctttt ctttaataat ctgaccaatg caacctcaca ctaagttagg 9420
ggaaagcaat aaagcagcaa gtctaaaata aactgaatag atgtgggccc ttccatcaaa 9480
gcaggaggca aagagtcccc cccctcaggc agctgtcagg aggtaaggag tgagtgtgcc 9540
tcaggggccc agccctcatc ttttcctccc cttcacttct cctaccctct tccatcatca 9600
ggagacctta gtcccatgca aagtcacaag aagtatccaa aacaaatttc agatgcaatt 9660
tcaaacgtag gacaaacata catgaaagaa gtagagaaaa tggaatagaa aatgagttca 9720
aacattctca ctgaatcttc ccagtaacat tgacagtctt ccctggaaat gggaggccat 9780
cttttctacc cctcctgtga ctctcatata ttttgctaaa caatgttttt gtcaaatgtc 9840
tcttcttttc aaggatttac tgatactcca caatttctaa aagtgagttt catggaacac 9900
tgatcacagg agtttccgtg gaaaagggtc ccatgagaaa ataagtttgg gaaacacttt 9960
aaactatatc tcccttctga agagaaacag cgtacactag tctattatag gtactaaaat 10020
gtctgtagta aagaaatctg tataatctgg tatttctcaa aatatttacc cactaaattg 10080
atttttttct gatgtgaaag atattaacag caaataaaat tggcctgtaa caaactgtag 10140
caattaatgt cctttacagc aagttcagac aacttggttt gtgttttcaa agccaagtca 10200
ggttccacaa atctgtcatc cttagcccct attatctctc agtatgaacc ttctaatcct 10260
gtaaggttgg cccaatcttt caaagaatgt ggctttatag ttacaaaata ttcatccaca 10320
ctaaccactg ttcagactca gatattagaa tctccctgca gaaaaactgt tcatcttagt 10380
cagacgtggt agcatgcacc tatagtccca gctactcagg aggctgagac aggaggatct 10440
cttgagccca ggaattgagg ctgcagtgca ctatgattgt gcctgtgaat agccactgcc 10500
ctccagccta ggcaacacag tgagaaccca tcccttaaga aagaaagaag gaggggagga 10560
agggaggaag gggggaaggg agggagggag ggaagaacta ctgatctggc atacatgtgc 10620
ttgatgaatc tcttcttctt tagaaaaata aaatggaaag aactcaggct tacaactagt 10680
catctctttt tccaaactct tcaaaagatg gttaataaga aagcatagct agaagaaatt 10740
ctaagcccaa acttcctcct aaactaggca tttcttttct aaaatccctt gtaatcaatg 10800
agtctccttt tgaaccctgc tcttggagag gaattcatta ccttttaagt caacacatcc 10860
catttctgga cacctctagt ggttaaagga gtttattctc atgtgtaaat aaaatctgcc 10920
tcttgaaact ctttgagaaa aaacagtata tttgggtcca atgaccaggg tgtgtgcagg 10980
agggacgtgg cacgcagtgc aggtaactct gctgtactgc ttatccagaa aggtgaatgc 11040

agtggcacaa aagcagctcg gccgcctgtg gcatacaagc accgtcttct tccaccctcc 11100
aatgcatgaa tatgcagaga agcttgccgc acttcttcct gagcctctta aggtacaatg 11160
ctcttcctgc aggaggtgaa tgttatgggg gaaaaaaaaa ggtatgatgc tcccagctca 11220
aagagacagg agtcttatga ttctagggga gcgtccctag gaagcaggga catgggcagg 11280
gaatttagcc tgtgcactga acatctgtag tggtgatctg atagctacat ctgaaagcaa 11340
tcagacagaa gaagggaatt tatgaattgc tggcgaaaca aaccaatgtt cccttgtgtc 11400
acctctgagc ctctgcctct ctttggttgc tctcttcacc atatcttagc agaaatcact 11460
tcccaggagc cgtaggcctc ttgatgttct catctatgtc ctcaagaggt ttttatctca 11520
tcgctcctta gcctttctgc aaagttattt gcatttcttt tacaaacagt gaaaccattt 11580
ttaataagtg ccatttggtt catttggtcc aaattacact tagatatttt tttctacatg 11640
agctgttttt ctgcatctga aatgactgat cttttaaatt gaactaagat gttaaaatgt 11700
tggccaagat ttccatttag acattaaacg agttaataca tgtgaagaac ctagaacaat 11760
gctcagtccc gagtgttatc tattattatt ttaccatgtt ctctacatta cctagaatag 11820
ataaagaaac atggaagagc tgcaacctca gttcagcccc agatgttcac agaagagcag 11880
aagcagagag tcagcagagt catgaacaca tgccttccag tccctgagtc tggagggcac 11940
agttctcttt cattctcccc attcttttgt ccaggctgag ctgcattttt tgtttacttg 12000
cttttttttt tctttttgag acggagtctc actctgtcac ccaggctgca gtgcaatggc 12060
gcaatctcgg ctcactgcaa tctctgcctc ctgggttcaa gtgattctcc tgcctcagcc 12120
ccccaagtag ctgggattac aggtgcccac caccacacct ggctaatttt tgtatcttta 12180
gtagagatgg ggtttcacca tgatggccag gctgatctca aactcctgac ctcaagtgat 12240
ccgcccgcct caacctccca aagtgctggg attacaggcg tgagccaccg cacccggctt 12300
gtttacttgt tttttcaaca ttcacacaca cagcatagtc aggctgggac aacccctgga 12360
gttgcagagt cagaggtcct gtcttgttcc aagcctttag aatcaggtga ctgggttaga 12420
gttaggagcc aagaaaccca acctttggat ctatcagcta ctcaccactg cgtgcccctc 12480
agcaagtctc ccttggcatt ttgcagacca tcaaataaag ccattaaaag ccaggaatgt 12540
tgcacagctg agacaatctc ttagccattt ttgtatgtct ggcacttagt ggctactgga 12600
ggaagggcta tggaattcat actgtggtta aatgatggaa ctgctgggta acccgagctg 12660
gtctgaaggg aactctcctt aatactcagg ggctggacac ctgcccttca gcaattttaa 12720
gtgaatgggt aataaggaaa taaattatga ggccacgtgt cttcctttcc tctccaggtc 12780
attttcttgg tgaacagtgg ctcagaagcc aatgagctgg ccatgctgat ggccagggcg 12840
cactcaaaca acatagacat catttctttc aggtaatcac aacttggaat attcaacttt 12900
aggaacactg acttaccccc attctaaatg tgtagcttta cattgtagca tggagtggga 12960
gagaggtttt ctcagggata tttttctagt tgttaagttt taaggaagga taggtcaatt 13020
gaattttatg acaggataac accattcaag ttggcctgat acaagagagg gggccccaac 13080

tggtcacgac cagcttgaag tcctcattat gtcatagatc ctcctgtgga cattaagaga 13140
aacccacatg acagtttttt aactttgaag atgtggcggg gaccacacca tataccaagt 13200
ggtaaatgac tgttccccac cttagaagat caaggctcta ccaaaaaata ccggcctatt 13260
ttaatttctg cttatgataa ggagctgtcc actgatttca tagtgccagc tgcacccatt 13320
ttttaccttt tattctcctt aagttcagag gatgaacata taaccctgta tatttctgct 13380
tctttcagtg acttttgagc actgataata gattcattaa taaaccctta atggctacat 13440
tagcactaaa aataatagaa aactatgcat tttgcttaca gttgcctccc ttgggtaatt 13500
gtatacagca tatttaaaca gactcctgta aagttaatgt aaaagacagg agtggcttat 13560
ggtaaaagtt gatggaatga ataaacagtt gcatttttaa gctgtctggt tataatttct 13620
gtgtgaattc aaaagaagat tttatttaat ccttatgaca gcacattcaa tactgcacat 13680
tccatatcaa atgagcatgc tgttattaca ggaataagaa atatgacaat aaagaattgt 13740
gattgatgta ttacctaaat gacattccag aatatgtgtg tcgaggtaag tattaagtag 13800
tcatggtact accagggttc aacacacatt ttgtctcctt tttcaagata cattaggtcg 13860
gtgcaaaagt aattgagatt tctgccatta aaagtaatgg caaaaaccac aattactttt 13920
gcaccaacct aatacaacca ttcttttgaa tagtcttaac ggtcctaaat ccttgtttaa 13980
aggtgatttt gatatgggaa attgttcgaa ctcatttgaa gcttacactg atgattagga 14040
gcatttatac taaatatgac tgtttcacgt tcaaaagaaa caaaacctgt gtttcttaga 14100
tggagaataa ggtgatttat aaggtgtgta agtccctaaa gatattgaac aacaggtata 14160
tcataggaaa aaatatttgg cttcccaaag cgactacttc gaagactggt attcattgaa 14220
catacaattt ctggtatgtt gttgctaaaa gatgagtctc attactatgg aataatagag 14280
tagggaaggt cctaagattg gagttcttgg acaaatgttc tccaaaattc ctttcacagt 14340
ttcaaatctg ttttctaaca acagttagaa aagactatta agtacattga tccgacataa 14400
gcttagatgc gttcttagaa atcagaggtg aatgcgaatg atgttctatt tcatcttcat 14460
agccttcctg ggttttctct tttgtcctac tcagtggacc aggaacttga ccccatcctc 14520
ctgttgtttc ttctctgcag aggagcctac catggatgca gtccttacac acttggcttg 14580
acaaacgtag ggacctacaa gatggaactc cctggtggga caggttgcca accagtgagt 14640
ttggagattt ttctcttgtt tctttaaaga ctgctgggta tgtgaagtag ttgatcaggt 14700
tttcctggta taaggattag aaactaatat aaagcctgtc attaatcagg ggctcaattt 14760
gacctaattg aattggagac cacttgagga caccattaga aaggccttcc gagtctgagt 14820
aataattaat gacagagttt taaaatgtat tttgctttta aataaaatca agtaaactgc 14880
aaggctctga gaatcttgaa tgcacaaaac ttctatgcag tcatcactgt actcaccttc 14940
tgttcccact tactgtaccc acattttcag catctgaatc agatacctaa ttattcttat 15000
tctttcagat gcccttaccc actgcaccct tgagcaagct gtttccaggc taacattgaa 15060
atcagtttcc tttgtagagg gcccacatag ctgatttcta agaaaaaatt atctcttcaa 15120
agcagatcct tcaattttca tcttaagtgg gggaagaatg agggaggaaa actaagaagc 15180

cctcttgtct tgatggcagc catatgctac cctaacttgg ctgtcttgtg ttcatgcttt 15240
gccacactcc ttcttttgtc atttgcagac aatgtgtcca gatgtttttc gtggcccttg 15300
gggaggaagc cactgtcgag attctccagt gcaaacaatc aggaagtgca gctgtgcacc 15360
aggtccgact gggcaagggg ggtgccagtg gagtgttgga agttggaagg gacacgaata 15420
aaggcagacc catccctgag ggaacaattg aatttgaaaa catggaaaag ggaaaagcca 15480
gaggaagcaa aactggaaag taaccaagtt attcctcatt gaatcaacgc ttatgtctgc 15540
tatcacttca ctctcagacc atctgtctgg ccatgtagga aaagcatctc acgctatcag 15600
agccaagctc aaaaaaacga ctatcagctg gtacttgatt caaggcattc tattagctct 15660
attataatag caatagctct ggtgtcaatt ttaaaagaca acaagctggg cgtggtggca 15720
tgcctgtagt cccagttact cgggaggctg aggtgggagg attgcttgag ctgaggaagt 15780
cgaggctgca gtgattcatg attatgccac tgcactccag cctgcatgaa agagcaaggc 15840
ccggcggggc atggtggctg acagctataa tcccagcact ttgggaggtt gaggcgggca 15900
gatcacctga ggtcgggagt ttgagaccag ctggccaaca tggtgaaatg gtgtctctat 15960
taaaaataca aaaattagct aggcatggta gtgtgcacct ataatcccag ctactcggga 16020
ggctgaggct caagaatcac ttgaacctgg gaggcagagg ttgcagtgaa ctgagataac 16080
gccactgcac tccaacctgg gcaacagaac aagactccat ctcaaaaaaa aaaaaaaaaa 16140
aaaaaaaaaa gcaagatccc atctctaaaa tgaaaaaact aaacaacaac caaaaaaaaa 16200
aaaaaaacaa gaaatcaaga tgggtgggat tattaaccca taccagtgga aagagggaaa 16260
ataatgcttt tggcaatctc aaaactccag tcaaaaagtt ctccagggac tgccagccaa 16320
aatgagagca tccgtcaaac aaaacacaag gctgcccttc ctacaggctg ctgacaaccc 16380
tggcaaaagt tttaaaggct ttctcacaag gagaacttcc atttaccaat cacattttta 16440
ttcgcattct ttacctacgc ttaacaggag ggagaggagg caggaaacat catctgttcc 16500
ttgctaatca acaagccctg cctctgattt gtcatcacaa cctttttaat agccgctcct 16560
tagtacttct gaaggaaata actaaatgct tcagcccagt gatttttaat ggaatagtat 16620
gaatcactgg gttgagagga aaaatacaaa acagaagtct taaaaaccag ctctgaaagg 16680
aaaaaaattc accatcgtct taaaattcag atgacataat agcaaaatgg aggtaagaga 16740
atgaaagagt aagtagtgaa agaagacaag atgtcacgct catggaattt aaaaccagaa 16800
ggaaatgtaa agctataaat caaactctcc actttaaaca tcaggaaagc cagtttcaca 16860
tggaggagac tggagagcta agattttacc ccaggtcttc tgactccaaa gccagtgctc 16920
ccaattccta tagccatcct catccacact gatgtgccgt caagataaac ccaagctgaa 16980
aaagcattcc tattccacag gccaggactg aggagctgca tgaagtcaag agatggattc 17040
cactctcagc cctggcatgg ttcagctatg agcaaaagct tcaccttttg cttcagtttt 17100
ctcatttgta aaacagagac agcaatatct ggctgctgcc aacaccatca cggcacttca 17160
tgagatgagg cacggagaac ttttgtagag aaaaacagac catagcaatg cctggtaaag 17220

ccgtatttga atattgcatc tgtaaggcat caagaactaa aatcagtttg atgcacctcc 17280
cagaccctgg tctgactgca aaaccaccag cacagagcct agtagacgga agcactaaaa 17340
ggtggtcatt tccttctttt cctgctatat gttcctcttt ccacatagaa gagacacaag 17400
cctactataa ggccttctat aggactactt aagccaattt gacaatacca gaaatccatg 17460
gtcatctctc tagtggcatc tagaggctaa atgatagtga caggcgtaca tttctgctct 17520
gaactgttgc ctttggcata tctgaagtca gttgttttct tttttattta tgtatttttt 17580
tttttgagac aaagtctcac tcttgtcccc cagactggag tgcaatggcg cgatctcggc 17640
tcactgcaac ctccgcctcc cgggttcaag cgattctcct gcctcagctt cccgagtagc 17700
ggggattaca ggtgcctgcc accacgccca gctaattttt gtatttttag tagagaccgg 17760
gtttcaccat gttggccagg ctggtctcga actcctgact tcaggtgatc cgctcgcctt 17820
ggcctcccaa agtgctggga tcacatgcgt gagccaccat gcccggccag ctgttttctt 17880
aactcacatc ttcaagggac aagactattt atggaatctt tgaacaggat tggaatacaa 17940
aagaaagacc ccagtctcct tcactgcctt taggacctgg gaagggtttg gtctttgctg 18000
taaaactgga atattcatat atgtttacaa aagcatccaa acatgtacag ttctttgact 18060
atagatgttg cctgttctca attcctctta acctattgat aggaatacct taagaggaat 18120
tgaggcacca accatggtag agatttttct catctcagaa atatgtcatc cctcccaaca 18180
gaggcagggg gccattttgt gagtctaatc tagatgttta tatggtatat ttatttgtcc 18240
tactccatct acctcccagg attttttccc caggggttca ttccaggccc tgagtttcaa 18300
gtgtaatcca tgacgagatt cactcctcat cacctactgc ccccacaatg ttccaaacct 18360
cgtactgatc atgtcattct tcttcttttg aaaggcaagt tttgtttttc ttaaaaattg 18420
tagttctttc ttggctcctc actcagctta actctttctg taccactagc tggaataaat 18480
tcttccttcg tcatcctgtt gtagtatcca acagaacatc ctgtctccac ccgcttgggg 18540
ttttcttcct gccaagcctc tgcagaagtg gcctaaccta gggttacatc aatgagtcaa 18600
tgcagctagt gcgaaataaa gtaaaaagct catcacagtc aatgactctc tcatcgctga 18660
gcatgtgggc agagagggaa aaaagtcatg aaaggtctag cataggttca ggcaaaacct 18720
tttggtttgg ttctgcccaa atttaagctc cactctttga tttctccagt tacaaatggg 18780
cttgttaaat ttccaaatta cttctcttga acataggtct gatccatctt ttagggtcat 18840
ctgaagctgt tttctcttcc tttcccaata aaaacgggaa atttctgtct ctagaggcat 18900
agatttattt gtctactata ttcttctcat caaaacaaca caagtgatgc caaaaacata 18960
ttttcagggt ttcatatact tgtcatttct tcctttctca cacccttaac ctccaacctc 19020
cagctaacca aagatatcag gtacctctgt caatcaaact tttccgaata tcacatgtaa 19080
tctaaaaata tgtttggggc cagctagtat ttcctcactt ttaatgaaga gctgaggtat 19140
aaaattcggt tttgatggtg attgcagaag gtttctgttc cttctgtcta gcaccgtgga 19200
gacagcactg catccatgcc tctaagcaag cactgggatg gtaggtgggg agccctgtcc 19260
cacactgctt tcactgtcac tcgggttgag gctttccatc ccttccacat ctttctctgc 19320

actcctttgg tcatctatgg atataaattt aagttcagag actgcatggt actgtttaga 19380
accaaataac aggtgtggat aaaaattctg aagattcagt catgtatgga tacagttgac 19440
ctgggacctt tgttattcca gagtagaaga atttctctct ctctctctct ctctctctct 19500
ctctctctct ctctctctct ctctctctct ctctcacctt tcctgcttcc ttggtgattc 19560
ccacttccaa ttctgacaca cagatgaagg cttctggagc ttgggcaagt ctgctgggca 19620
atcttcccac tcatctctag tcctctccca tccacaggat ttcatgcctg tgtgtctgtg 19680
tggtgaggct tgcagctagt gaagcatggc agaaatgttt ctagtatctt taagcaggat 19740
tagaaagtag acatggttag tgctaagaaa actgagctgg atggacagct agtactggta 19800
acatgatagc ctgggttttc tccagtggaa aacaactaaa aaatcctgga taaaatgaaa 19860
tgcattccct tcaaagcatg gatacaagaa agcacgaatt ctcagaaacg caaatagatc 19920
attacggcac tgcagccccc tttgaccctg agagaatttc ttgaacctgg taaccttgag 19980
cttccatgtt gatggctctg tggagtgcag ggaagagatg acgaagtcca gggcccagcc 20040
aaagtaaggg atcttatagg aaaccctccc ctgccacaaa gctggagccc ccaagaggcc 20100
acatcttcat ccatgggtca actagaatta aatacaccac caagaacata ttacccctca 20160
caggccaaag ggcctgcaag gattctggtg ctgaatggaa gtggagggac tgggaggagc 20220
caccactgaa agtttcacct gcagactggc cctccaaggc tttgcattgt gaacgctcac 20280
ttcctcaagc tcataattta aaggactctt agactctaat gccctacgca cttggaagct 20340
gcaaactcag ataggctctg gagggatcta cctcattctg ggcctcaaag aattctcaca 20400
aatgaaattc caaggaaaat aaactgcccc ttgtcaaaaa taaatataca atacacaaaa 20460
ctccgcaatc aagaaaaaga tcagcaagga tttcaaataa cagaattatc agataggcta 20520
taaaatgaat atgtttaata catttaaaga aaatcaaata taagcttgaa aatatgcgaa 20580
agggatagga cactgtgaag aataagaaag caagttttaa aagcaaatat gccttctagt 20640
aatataaaag tgattattga aatttaaaac tcagcatgtg ggtttatcag cagtaagatg 20700
cagccaaaag gagaagtaat gaattaaaga cagatcactt caacaaggac actttactgc 20760
ttttccccat tagaaagatc tccaaaaaga aagaaagaaa gaaaggcctt caagcaaaat 20820
tgctcaatgt ctcactccag ccatgatttc tactatttgt taatattttc ttcttaatgc 20880
gttaaacatt tactgagcac cacctacagt gtaccaaaca gcttgcctgg ccacagagat 20940
tttagaaatg aacaaagtat agtccctgcc cacaaaaacc tcctgattaa tcggagaaag 21000
tcctgttaaa aacaagcaca ggaccaagtg ccaagggctg cgatggatgg aagccaccta 21060
gctctcgaac atttctcttt agactgaatt ctaattgttc tacaagcata tttactaact 21120
ctaaagacaa agacactttg tagctttaaa acggtacatc agattaaatg gcaatgaaag 21180
gtaaatagag ttattttcaa ataaattaaa tcgctctaat tacatttgaa ttagatccac 21240
ctgtttgaga ggacgggcat ccttaggtga aacagctatc tataccagct caatgacact 21300
ttgggacgtt tcttccccag atggaaagaa ctgaagtggt ttgtttatgc ctttatggct 21360

atcttaatct ccctccttga accaaagtac aagttgttca gagagaacat tcccccttca 21420
gaaatgcata tcctcacccc agtttaccgt attctgaagt ccctgattaa cttaccctgt 21480
ctggttgttt tttttttttc ctgtttttcc ccatgtttct aaatatcagg ctcacatttt 21540
caaatgctgt ggtttgtttt gttttgttgt ttgctttttg cagactgctg ccaagctaaa 21600
gatcagtata ttgagcaatt caaagatacg ctgagcacat ctgtggccaa gtcaattgct 21660
ggatttttcg cagaacctat tcaagtgggt atatgaacct tagacattaa tggagctgaa 21720
tcttcaatta tcagaaagtt ttaactcata tattctagag aattccaaat gaattaagtg 21780
ctcactgtgt ccctctctaa attcattctc ttggtctcct tgaaaaaaga agacgctgac 21840
tttcaaatga ccttttaaaa accctctcct ccattctcac cagacagatg aaactaccga 21900
actcctaaaa ttcttaagaa gaggaaaccc tggccatttt tagctgctta ataacatccg 21960
cattgtccaa agaactcttt ctttagtctc accaatttct tttattctac tgttttctaa 22020
tctcagaaaa taatcaatgt ttgcatcttt tttccagaag tcatggtata gagtaataag 22080
tcctcttttc acccacagtg gaaactgttc tacagactgt tgttaaaatt gctagtgtta 22140
ctgtcatgat aatgatgata atgatgatct aactttttta tatactttga aagggctgat 22200
gctattatta taggtcttct tggtctggtc ttttgcaggg tgtgaatgga gttgtccagt 22260
acccaaaggg gtttctaaag gaagcctttg agctggtgcg agcaagggga ggcgtgtgca 22320
ttgcagatga agtaagtggc atgtaagggt gccattgagt gcagtgggaa caggagatgc 22380
acagacagaa acctcctaaa cttcctctgt gttccaggca gaaagcctct gctctgtgaa 22440
ttccaaagtt tgcctacaaa tgaatgaaat ttgtgctagt gggctggttg atttgttagt 22500
acattcgctg atcagtttaa caagtataac agaacttaaa tctccaaatt gatgtctatc 22560
aaataagttg ctaacagcca tatgtgatgt tattagaccg tgactcaata tattttttta 22620
attcctttct agggattgtt tcaggggctg acaaacatca taggaaattt aatctcatag 22680
ccaaggtttt tgttgttttt atttgtttgg ttattttgag acaaagtctc gctctgtcat 22740
ccaggctgaa gtacagtggt acaatctcga ctcaccgcaa cctccgcctc ctggggttca 22800
agggattctt ctgccacagc ctcccgagta gctgggacca caggcatgtg ccaccaagcc 22860
tgactaattt ttgtattttt agtaaagaca gggtttcacc atgttggcca ggctgatctt 22920
gaattcctga cctcaagtga tccgcccgcc ttggcctccc aaagtactgg cattagagac 22980
gtgagccacc gtgccaggcc tgatagccaa gtttttaatc tttatttttg caaaaatagt 23040
atttaccaac ctggcagcat atacctttct gacaaaactt gcctggaaat aatgtgggtt 23100
tttttgtttg tttgtttctt tttgagacag aatttctttc ttgttgccca ggctggagtg 23160
cagtggtgca atctcagctc accacagcct ctgcctccca ggttcaagtg attctcctgc 23220
ctcagcctcc cgagtagctg ggattacagg catccgccac cacacccggc tagttttgta 23280
ttttttagta gagactgggg tttctccgtg ttggtcaggc tggtctcgaa ctcctgacct 23340
caggtgatct gcccaccttg gcgtcccaaa gtgctgggtt tacaggcatg agccaccgcg 23400
cctggccgaa ataatgtttt taaaataata ataataatcc agcctctaaa ttagaggagt 23460

accaccaact gcaggtactg catttgaaaa tagttcccca aaaattgtaa aataggttga 23520
attaaagcag cattaaagca ttaaagcaca ctactcccaa gggcattact ttaatgggaa 23580
gaaaattcgt tggctatgtg agctctagcc tgtgtgtttt caaaagggat taatttactt 23640
ataatcacac ctcatattag agtgtatcaa ataaagcagt cctctgtcta ctgagagtag 23700
ctatggagat taaaagacca gaggatgatc actagggcca ccgaagccat gtctcactgc 23760
tgggtgacta tcatgattgg actaacaggt ggttagacca gccaaggaca ggtggagaac 23820
ctgtaagtat gtttcacacc tatttctgtc cagccatcat tgctggaata gacttcctca 23880
actaaaaatt tcctacagct aggttgggcg cagtggctca catctgtcat cccagcactt 23940
tgggatgctg aggcaggccg gtcacttgag gtcaggagtt caaaaccagc ccagccaaca 24000
tggtgaaaca ccatctgtac taaaaataca aaaagtagct gggtgtggta gtgcatgcct 24060
gtaatcccag ctgctcagga cgctgacgca ggagaatgtt tgaacccagg aggtggaggt 24120
tgcaatgagc caagatcatg ccattgcact ccagcctggg caacggagca aaactccatc 24180
tcaaaataaa taaataaata aataaataaa taaataaata aataaataaa tattccctac 24240
agctgacact gagctgtcac atcagcagaa attttgcaat ttctgcctgg gcagctgggt 24300
ctctgttatg cccaggtgta ttggatgtcc ctggtgtacc acaggcaact tgggccagcc 24360
tgctcctgct gagaccatcc tcccttctcc aatctgccca caaactgatc gaagtccagg 24420
ctggagaaag ttaatctgct actttaaaaa aatgagtata tccagtcagc ttgaaattag 24480
ctacatcagt aaaaaggata aactttggcc tccaaatcag cacatagtga agctgtcaca 24540
gtcagactaa ttaaagagaa ggtaggctga attcaaagga aatatgaatt tcaaaatttt 24600
ctgtcttgtg gttttatgac tttcaaacaa tgtaattgaa aactatctaa cacaggcaat 24660
cctcaagaag gactatcgcg aggactaggt aaaaataaga atagctggaa ctctctgagt 24720
acctgctatg tgtcagacac tgttcataac actttgcatg tgttaactca gttcatacct 24780
atcaactgtt ctatagtatg ccctgttgct gctgttccca cgtattagtc ctttctcatg 24840
ctgctgataa agaaatacct gagactgggt aatttataaa gaaaaagagg tttttctttt 24900
ttcttttttc tttttctttt ttttttttac atctgccatc accacaattt tattaaaaat 24960
gaagcaaata agacaaagaa tatgtgaaac ctatacaaac aaatgaataa aacaatatcg 25020
gaataaaagg cactctttta aattgaaaat agttttgtaa agtaaactca ctaattaaaa 25080
aaaaaaattt gcagaagaca aacatttagg agccagtgat tgaaatcacc aagactcttt 25140
tatgctaact ttcccttttt gggcgtcaat tactactctg ccttttctag tttacagtat 25200
tcttcataga aaaatgtaga agaaaaacaa tattatcaga gttctgtctc cctctggtac 25260
ttggttaata ttatagcata ttctaaaggg aagggctcta ggttttcctt gtgctttttc 25320
ttttcttttc cttttttttt ttttttactt ctctcttttt atttattatt attatacttt 25380
aagttttagg gtacatgtgc acaatgtgca ggttagttgc atatgtatac atgtgccatg 25440
ctggtgcgct gcacccacta actcgtcatc tagcattagg catatgtccc aatgctatcc 25500

ctcccccctc cccccacccc acaacagtcc ctagactgtg atgctcccct tcctgtgtcc 25560
acgtgttctc attgttcaat tcccacctat gagtgagaat atgcggtgct tggttttttg 25620
ttcttgcgat attttactga gaatgattat ttccaatttc atccatgtcc cttcaaagga 25680
catgaactca tcatttttta tggctgcata gtattccatg gtgtatatgt gccacatttt 25740
cttaatccag tctatcattg ttggacattt gggttggttc caagtctttg ctattgtgaa 25800
tagtgccaca ataaacatac gtgtgcatgt gtctttatag cagcgtgatt tatagtcctt 25860
tgggtatata cccagtaatg ggatggctgg gtcaatggta tttctagttc tagatccctg 25920
aggaatcacc acactgactt ccacaatggt tgaactagtt tacagtccca ccaacagtgt 25980
aaaagtgttc ctatttctcc acatcctctc cagcacctgt tgtttcctga ctttttaatg 26040
atcaccattc taactggtgt gagatggtat ctcattgtgg ttttgatttg catttctctg 26100
atggccagtg atgatgagca ttttttcatg tgtttgttgg ctgcataaat gtcttctttt 26160
gagaagtgtc tgttcatatc cttcacccaa ttgttgatgg ggtagtttgt ttttttcttg 26220
taaatttgtt agagttcatt gtagattctg gatattagcc ctttgtcaga tgagtaggtt 26280
gcgaaaattt tctcccgttt tgtaggttgc ctgttcactc tgatggtagt ttcttttgct 26340
gtgcagaagc tctttagttt aattagatcc catttgtcaa ttttggcttt tgttgccatt 26400
gcttttggcg ttttagacat gaagtccttg cccatgcctg tgtcctgaat ggtaatgcct 26460
aggtttcttt ctagggtttt tatggtttta ggtctaacgt ttaagtcttt aatccatctt 26520
gaattgattt ttgtataagg tgtaaggaag ggatccagtt tcagctttct acatatggct 26580
agccagtttt cccagcacca tttattaaat agggaatcct ttccccattg cttgtttttc 26640
tcaggtttgt caaagatcag atagttgtag atatgcagca ttatttctga gggctctgtt 26700
ctgttccatt gatctatatc tctgttttgg taccagtacc atgctgtttt ggttactgta 26760
gccttgtagt atagtttgaa gtcaggtagt gtgatgcctc cagctttgtt cttttggctt 26820
aggattcact tggcgaagcg ggctcttctt tggttccata tgaactttga agtagttttt 26880
tcaattctgt gaagaaagtc attggtagct tgatggggat ggcattgaat ctgtaaatta 26940
ccttgggcag tgtggccatt ttcacgatat tgattcttcc tgcccatgag catggaatat 27000
tcttccattt gtttgtatct tcttttattt ccttgagcag tggtttgtag ttctccttga 27060
agaggtcctt cacatccctt gtaagttgga ctcctaggta ttttattctc tttgaagcaa 27120
ttgtgaatgg gaattcactc atgatttggc tctctgtttg tctgttattg gtgtataaga 27180
atgcttgtga tttttgtaca ttgattttgt atcctgagac tttgctgaag ttgcttatca 27240
gcttaaggag attttgggct gagacaatgg ggttttctag atatacaatc atgtcgtctg 27300
caaacaggga caatttgact tcctcttttc ctattgaata ccctttattt ccttctccta 27360
cctaattgcc ctggccagaa cttccaacac tatgttgaat aggagtggtg agcgagggca 27420
tccctgtctt atgccagttt tcaaagggaa tgcttccagt ttttgcccat tcagtatgat 27480
attggctgtg ggtttgtcat agatacctct tattattttg aaatacgtcc catcaatacc 27540
taatttattg agagttttta gcatgaaggg ttgttgaatt ttgtcaaagg ccttttctgc 27600

atctattgag ataatcatgt ggtttttgtc tttggttctg cttatatgct ggattacatt 27660
tattgatttg catatattga accagccttg catcccaggg atgaagccct cttgatcatg 27720
gtggataagc tttttgatgt gctgctggat tcgttttgcc agtattttat tgaggatttt 27780
tgcatcaatg ttcatcaagg atattggtct gaaattctct tttttggttg tgtctctgcc 27840
cggctttggt atcaggatga tgctggcctc ataaaatgag ttagggagga ttccctcttt 27900
ttctgttgat tggaatagtt tcagaaggaa tggtaccagt tcctccttgt acctctggta 27960
gaattccgct gtgaatcctt ctggtcctgg actctttttg gttggtaagc tattgattat 28020
tgccacaatt tcagatcctg ttattggtct attcagagat tcaatttctt cctggtttag 28080
tcttgggaga gtgtatgtgt tgaggaattt atccatttct tctagatttt ctagtttatt 28140
tgcatagagg tggttgtagt attctctgat ggtagtttgt atttctgtgg gatcagtggt 28200
gatatcccct ttatcatttt ttattgcatc tatttgattc ttctctcttt ttttctttat 28260
tagtcttgct agtggtctat caattttgtt gatcctttca gaaaaccagc tcctggattc 28320
attaattttt tgaagggttt tttgtgtctc tatttccttc agttctgttg attttagtta 28380
tttcttgcct tcttctagct tttgaatgtg ttttctcttg cttttctagt tcttttaatt 28440
gtgatgttag ggtgtcaatt ttggatcttt cctgctttct cttgtgggca tttagtgcta 28500
taagtttccc tctacacact gctttgaatg cgtcccagag attctggtat gttttgtctt 28560
tgttctcaat ggtttcaaag aacatcttta tttctgcctt catttcgtta tgtacccagt 28620
agtcattcag gagcaggttg ttcagtttcc atgtagttga gcggttttga gtgagattct 28680
taatcctgag ttctagtttg attgcactgt ggtctgagag atagtttgtt ataatttctg 28740
ttcttttaca tttgctgagg agagctttac ttccaagtat gtggtcaatt tttgaatagg 28800
tgtggtgtgg tgctgaaaaa aatgtatatt ctgttgattt ggggtggaga gttctgtaga 28860
tgtctattag gtccgcttgg tgcagagctg agttcaattc ctgggtatcc ttgttgactt 28920
tctgtctcat tgatctgtct aatgttgaca gtggggtgtt aaagtctccc attattaatg 28980
tgtgggagtc taagtctctt tgtaggtcac tcaggacttg ctttatgaat ctgggtgctc 29040
ctgtattggg tgcatatata tttaggatag ttagctcttg ttgttgaatt gatcccttta 29100
ccattatgta atggtcttct ttgtctcttt tgatctttgt tggtttaaag cctgttttat 29160
cagagactag gattgcaacc cctgcctttt tttgttttct atttgcttgg tagatcttcc 29220
tccatccttt tattttgagc ctatgtgtgt ctctgcacgt gagatgggtt tcctgaatac 29280
agcacactga tgggtcttga ctctttatcc aatttgccag tctgtgtctt ttaattggag 29340
catttagtcc atttacattt aaagttaata ttgttatgtg tgaatttgat cctgtcatta 29400
tgatgttagc tggttatttt gctcgttagt tgatgcagtt tcttcctagt ctcgatggtc 29460
tttacatttt ggcatgattt tgcagcagct ggtaccagtt gttcctttcc atgtttagtg 29520
cttccttcag gagctctttt agggcaggcc tggtggtgac aaaatctctc agcatttgct 29580
tgtttgtaaa gtattttatt tctctttcac ttatgaagct tagcttggct ggatatgaaa 29640

ttctgggttt aaaattcttt tctttaagaa cgttgaatat tggcccccac tctcttctgg 29700
cttgtagagt ttctgccgat agatctgctg ttagtctgat gggcttccct ttgagggtaa 29760
cctgaccttt ctctcttgct gcctttaaca tttttccttt catttcaact ttgatgaatc 29820
tgacaattgt gtgtcttgga gttgctcttc tcaaggagta tctttgtgac attctctgta 29880
tttcctgaat ctgaatgttg gcctgccttg ctagattggg gaagttctcc tggataatat 29940
cctgcagagt gttctccaac ttggttccat tctccctgtc actttcaggt acaccaatca 30000
gacgtagatt tggtcttttc acatagtccc atatttcttg gaggctttgc tcgtttcttt 30060
ttattctttt ttctctaaac ttcccttctg tcttcatttc attcatttca ccttccatcg 30120
ctgataccct ttcttccagt tgatctcatc agctccggag gcttctgcat tcttcacgta 30180
gttctcgagc cttggttttc agctccatca gctcctttaa gcacttctct ttattggtta 30240
ttctagatat acattcttct aaattttttt caaagttttc aacttctttg cctttggttt 30300
gaatgtcctc tcatagcttg gagtaatttg atcgtctgaa gccttcttct ctcaactcgt 30360
caaagtcatt ctccgtccag ctttgttccg ttactggtga ggaactgcgt tcctttggag 30420
gaggagaggt gctctgcttt ttagagtttc cagtttaaga aaaagaggtt taaaggacag 30480
agttccatgt ggttggggag gcctaacaat catggcggaa tgcagaaggc acatcttaca 30540
tggtagcaga caagagacaa tgagagtcaa gcaaaaaggg tttcccctta taaaaccatc 30600
agatcttgtg agacttattc accaccatga gaacagtatg gggaaccacc ccccatgatt 30660
caattatctc ccatcaggtc cctcccacaa cacgtgggaa ttatgggagc tacaattcat 30720
gatgagattt gggtggggac acagccaaac catatcatct cattttaaat ataagtgaat 30780
gtaggtccaa ggtggttaaa caacctgtca agcttatgcc gttagtagga ataggtccaa 30840
gactcaaact caggctccca aaaccacatg actagccacc gtatcaagct gcccctccaa 30900
aatgacagag cgtggtgtag aaagggcaaa tcatggcaga aagggaagga acagaggaca 30960
gaaaaacctg agtgcagagg gccagttctg tcaaggagca gtcttacaca agtcatctta 31020
attaacaatc agtggcattc ttttctttaa aatgtggaat ctggattaaa gttagaaaac 31080
tgaaacctct gggcccatat tggcatagac tgattttgaa tttgaaactg actgcaagac 31140
atgcataccc gtagtcccca ccccacttgc tgttgtgacc cagcctgctt ctgtccctaa 31200
catatgcttc tcatctccaa agacagttga gtttgtggac cttagaatga tccatctctg 31260
tactactgta agagtctatg aaactgatac ttcctcagtg actaaacatg gtttatggtt 31320
ttacataatt ccatgcattt gtaactgagt ccaaagcttg taccacttgc tgcaaaacag 31380
tcagtaagtc aagcaataag atgttggggc aaggaagacc actttatttg ggggagcaag 31440
caaaccgaga agacggcagg ctcatgtcct aaagaaccac cttaagcata tgtgggttcc 31500
ttttatgtta agggaaaggg gaagagggag ggggttgagg tcaaaaggtg actggtgacc 31560
acagacgttt gggctgcagc aaggataaac tttcatcctt ggtcaggtta caatactgtt 31620
ataaattttt aatgtaatgt tgttacttgt gtgttcatcc ttcttatctc ctcaggattt 31680
ctttttaaag catccttgcc tcaaagttaa accataaact aaattcctcc catagttagc 31740

ttagcctata tgcagagata aacaaaagca gttaacctaa aagacatcat cacagtggga 31800
ggatgagagc aagctggtta ggagcaaaat ggattaagtc atgctaggct tccttttcac 31860
tgttacacat tgtccaagca atctgatttt tcaccattta tgccatttat gtatggtatt 31920
tagatggagt gaaaaagaaa gacacgcagg aagacaaaat ggatggattt tccacgagct 31980
cctcgtacat tatcccattt ggaaaaatca atcagctgcc cagaggctca ttagtaggaa 32040
ggaggcaacc caggattcca gcccaggtct gtctgactcc cagattaagc tctgattcca 32100
gcccaggtct gtctgactcc caggttaagc tcttctccta cactggtcac tatcatctaa 32160
ttctaacccc tggaatctgt ccttgaaaat gagaaaggaa tctgaaaagg aggctcactc 32220
tttttttttt tttttttttt ttttgagatg gagtctcact ctgttgccca ggctggagtg 32280
cagtggcatg atcccggctc actgcaacct ctgcctccca ggttcaagcg attctcctcc 32340
ctcaacctcc caggtagctg ggactacagg ctcccgccac cacactcaga caatttttgt 32400
atttttagta gagatgaggt ttcatcatgt tggccaggct ggtctcaaac tccggacctc 32460
aggtgatcca cccacctcgg cctcccaaag tgctgagatt acaggcatga gccaccgtgc 32520
ccagcctgga agctcactct tttaagtgca tttcagaatc ggcttctagt gcccacatcc 32580
ctaaccccca ttttgcattt tgtgtatgtg tggagagaga cagacagaca gagagacagg 32640
cagggagact gagagccaga aagaaatcga ggagacttca ccagaaaggc tctggcagaa 32700
atgtgcgtgg agaagtttgt atggctttat ttaatcaggc aattctgcca ccctgtcatt 32760
cattccacaa gtgcttacta agtgtctact ttgtatcagg cactgtgcta ggtgttatgg 32820
atacagaatg gaataagaca cagtccctgc catcggacat gcccactggt gataacagtc 32880
ctctatgaaa gtctctatgg cagccttccc agagtacctt gagagggagc ctaactaagg 32940
ctcaggagaa ccttcagaaa ccaagtgatg gcaaagtttg ttttgaagca cagaagaagt 33000
cggtgagaca aagcagcaag aagagggtct tctagaaaga gcgaacagca tgcacaaaaa 33060
aactaaggtc aacttttagt ccaaaccaca attcctaaat tagaggagtt caaccctgag 33120
ttaactgcct cgtctgggtc ctcctcatga tacagttctc aggaagggga aggcatccct 33180
gggaagagga tgaccagttt aactgggctg gagcaagtgc cccagctcca cttgactgac 33240
agtaactccc tcttcatcta tcctggggca gactcagctt ctctactttt ctatcctgct 33300
ttcttacaca gaggaaacgc tcatcaaaga ttgttaatca taatgatccc tgtcataata 33360
tttgtatgac cctcatggag cctgtgacat attgttgtat ttctacttga cggatgagtt 33420
agcagaggtt ccaaggactt aagaaacata cccagaattg cacaaagcta ggaaatgact 33480
cttgatgcct aatagctcag gcacaaatta agcgctttgt ccatgatact acaggatgct 33540
catgataatg gctgatgatg gccatcatga ggatgaggag aaagaagaaa gggaagagaa 33600
cagagaagaa aacagcattt cttttttttt tttttttttt tgagatggag tctcaccctg 33660
tcactcaggc tggagtgcag tggtgcaatc tcggctcact gcagcctctg ccctccgagt 33720
tcaagcgatt ctcctgtctc agcctcccga gtagctggga ttacaggcac ctgccactgt 33780

gcccggctaa ttttttgtat ttttagaaga gacagggttt caccatcttg gccaggctgg 33840
tcttgaactc ctgacctcat gatccacctg cttcagcctc ctaaagtgct gggattacag 33900
gtgtgagcca ccgcgccctg tcgaaaacag catttcttga agggtttcca atggttttga 33960
acttgttttc ccaggtgcag acaggatttg gaaggttggg ctctcacttc tggggcttcc 34020
aaacccacga tgtcctgcct gacattgtca ccatggctaa agggattggg aatggctttc 34080
ccatggcagc agtcataacc actccaggta ggacccacag gcagtttgtg tttgcttctg 34140
ggcctcgtac attgggctat ggtataacac gtgtgttgga acttgtgtag attggagaca 34200
atcatggaaa ccacatgatg catttcccaa gcaaatgaaa gagagaaaca gtctgtctgc 34260
ctgactgtga aacagttgga agaaagccag cccttttttt tttttttttt tgagacagag 34320
tcttgctctg tcacccaggc ttgagtgcag tggcacaatc ttggctcatt gcaccctctg 34380
cctccgcagg tgcaagcaat tctcctgcct cagcctcctg agtagctgcg attacaggtg 34440
cgcaccacca cgcccagcta atttttgtat ttttagtaga aacggagttt caccatgttg 34500
gccaggctgg tctcaaactc ctgacctcag gtgtttgacc ttggcctccc aaagtgatgg 34560
gattacaggc gtgagccact gcacccctat ccgaaagcca gcccattctg atctacctgt 34620
agggaagact aattctccca tgcctatgat gaaactcagg gttccaccac acttttgagg 34680
tatatggaag agggagagaa gatggaaaga ggagaggggt gggagagggc tctggtgtct 34740
cttcctcttc ttataagaaa accagttcct tgggcttaga tagggcccca ccctgatgac 34800
cttatttaac ctaattacct acttaaacgc actatctcca aatacagtct tattcagggt 34860
taaggcttca gccatataaa ttttgaggcg atacaattca gtccctaaca tctactatca 34920
gttgaatgaa ggatgaattg gattggatgc catttacaac acgaagtaac tgtagtccaa 34980
atggcgcgat gagagattgt ggacaggaat gtgtccctct ttccacacct tctattttct 35040
ccctctagag attgccaaat ctttggcgaa atgcctgcag cacttcaaca cctttggagg 35100
gaaccccatg gcctgtgcca ttggatctgc tgtgcttgag gttggttcca gcggccacat 35160
taacctcact catttcacaa atgactcttt caaaaccaca catgcattag cagaactcta 35220
tcatgctgag ttgttaaagg ggagagttaa ttcatagttt atccattaaa gttagatttc 35280
catttattat tctgccttct ccctttttaa agacctactt tgggtgctga aagtattgga 35340
taagaaataa tatataataa acctcacata catgggaaat ataagaattt tcctagccag 35400
ctgaaatttc tttgttatct tggtagtgat ttatagatgt taactgctat ccttgtattt 35460
agtttcatgt tggccttctc ttgtgtattt tttcttcctt ccttttgttg tactattata 35520
aattgaatga ctaaggagac actgagttct acgtgcttac agcaaggtaa ttcagctgtt 35580
cacccctaca ccattgattt agcctgggat cgcatctgtt taaaaaaaga ttttttttaa 35640
tgttcttgtt aacacgccta atatcatttg cttaaagaac tgtaacaaat ttctggaaga 35700
atgtctaaga agtaaatgat ggtttttttt gtggggagga aaactaggtg gctgggggcc 35760
aagggtcaac aagggtcatt ctgctgtata tacttttata cttgctgata tttatatata 35820
tacatacaca tatatattta tatatgtata tatatttatt tatttcaata gcttttggga 35880

tacaagtcgg ttttggttac atggatgaat tgtatagtgg tgaagtctga gactttagtg 35940
cactgtcacc caagtagtgt acattgtacc caatatgtaa ttttccatcc ctcacccccc 36000
tcccagcttc cccgcttctg ggtctccaat gtctattgtg tacccatagc ttagctctca 36060
cctataagtg agaacactta ttagttgctg tgaaagacat tattttcttt tttatggctg 36120
agtggtattc catggcgtgt gtgtgtgtgt gtgtgtgtgt gtgtgtgtgt gtgtgtgtat 36180
acaccacatt ttctttatcc actcatcaat tgatgggctc ttaggttggt tccatatctt 36240
tgcaattgtg agttgtacta caataaacat atatgtgcag gtgtcttttt gatataatgg 36300
cttttttttc ctttgagtag atacccagaa gtgggattgc tggaccgaag gatagaacta 36360
cttttagttc ttttagaaat ctccatactg ttttccatag aggttgtact aatttacagt 36420
cccactagca gttacactca ctgattttcg accatgaact tgatttacct attccaaaaa 36480
aattttaaat aaaaataaat aagaaaacac tgcctttatt aattaaatag ttattttcaa 36540
ctacaattaa gtcatgagat gagaaagtat gggtaggaat gtgtcattta tttcactttg 36600
tagacattgc caattttttt tttttttttt tttttagaaa gagtcttgct ctgtcaccca 36660
gactggagtg caggggcaca actttggctc actgcaacct ctgcctccca ggttcaagcg 36720
attctcatgc ctcagcatcc caagtagctg ggattatagg catgcaccac catgcccaac 36780
taacttttgt atttttagta gagatggggt ttcaccatgt tggccaagct agtctcaaac 36840
tcctaacctc aagtgatcca cccacctcag cctcccaaag tgctggtatt acaagcatga 36900
gctgacattg ccaacttctt ttaaaaagtg ctttgttacc tcaaccaatt tagaaggcaa 36960
gaaggtttag gtgaacaaat aggtctataa aaataacaag gtttgcctgg agtcatgcaa 37020
ttattgtcag ctaaataaaa atatagtata tttcatgtat cttgtcaaga accacaggtg 37080
tttgctgtaa gtaaaactga ggtaaaaatc cttttatatc ttctggtgcc ctggctctgg 37140
gctcagccaa agaggccaga gcctcagacc aggctgcttg gctatgagag tccatgtctg 37200
gctgaggcct gctgagcaaa ggtcaccatc ttccagtagg gaaaccatct gtgatatttc 37260
tttccacaga gcattcatct tattcacaaa tatatcccag ttctcagcaa agcctgacaa 37320
taacaagcct tgggatttca gaatctacat tttcaactgc attgagacac acctttattc 37380
tttcctaaat tcttttttgt ttgttttttg aggcaaagtg tcgctctgtc acccaggctg 37440
gagtgcagtg gcgcgatctt ggctcactgc aacctcctcc tcccggattc aagcaattct 37500
cctgcctcag cctcccgaat agctgggact acaggcgccc cccacagagc cctgctaatt 37560
tttgtatttt ttttttaaag tagagacggg gtttcaccct attggccagg ctggtcccga 37620
actcctgacc ttgtgatcca cccatcttgg cctcccaaag tgctgggatt acaggtgtga 37680
gccaccatgc ctggcctatt atttcctaaa tacttagcca cctctgaagg tgagggagtt 37740
gggggcagaa gcatatcccc tcccctaaga cttttcttct ctccttacaa cagatctttt 37800
tgtagtcctt gtggtcacac tagacttgtt ctgcattctg tttggttaag ttacaagagg 37860
gccatgtcaa atgacttctc cacgattctc agtcaatctt agaacttctg ccatgtaaga 37920

tcatttgggg ctttctctta acaggtgatt aaagaagaaa atctacagga aaacagtcaa 37980
gaagttggga cctacatgtt actaaagttt gctaagctgc gggatgaatt tgaaattgtt 38040
ggagacgtcc gaggcaaagg tctcatgata ggcatagaaa tggtgcagga taaggtaggt 38100
gctaatctgc ccctctctct cagctgcttg gagccagcag gagatatagg aggaaacatc 38160
agctaaactt catttactct caaggagaga gcagacacaa acagcaagat ttcagcaaag 38220
ccctttagag attagtagag agtgttgagt atgaacaaaa tgctggcaga tcatcaagaa 38280
ttctagaggg atgagggaga gcactgggaa aagaaaccaa cattatcctt gccatcctag 38340
gcaaatagca taggcaatga ccgaatactt agaaatacag ggaagggagg aaattatctg 38400
gggagaaaca gaaggaataa gaaagaaggg ccaggaaaat gttatggtga aggtttgggg 38460
tgaggaggtt aaatatatat taggagttgt tgttgttgag acagagtctc actctgtcac 38520
ccaggctggg agtgcagtgg tacgatcttg gctcactgca acctccacct tacaggttca 38580
agctattctc gagcctcagc ctcccgggta gctgggatta caggcacaca ccaccatacc 38640
tggctaattt ttgtattttt agtagaaatg gggttttgcc atattgccca ggctggtctc 38700
aaactcctta cctcaagtta tctgcccacc gtggcctcct gaagtactaa gattacaggc 38760
atgagccacc aagcctggcc tatattgagt tttgtttgtt tgcatgctta cttgtgaatg 38820
gatttcaacc tcccaagtaa ccagggagga atgttgttac ttgtgaatgg atttcaacct 38880
cccaagtaac cagggaggaa tgttgcccac tgagacctct aactcatcat aacagcaatt 38940
gaacttagca tcttccccca aaccagatat ttctcccaag atcactgttt gtttctcaca 39000
ctccaccgtt ctcccaatta ctcagcctca atagcacaaa atcatctgac tcctcctctt 39060
cccttgtttc taacaacagg actcattgat atctcttctc tcccatgtct ctcaaatctt 39120
tccctttgtt tctactgcag cccctatagt aaatgcctgt aggaaattgt gcctttcctc 39180
cataaaatca aagcctgaaa agagcaggag aatcttggga gtagggagga atagccagac 39240
taacctcatg tctacaggga tccagctttc cctagcaatt tctacctctt tggttttacc 39300
accattctac tttagaagga ttgatgctca attttttaga ccagacatgg tataagactc 39360
ttcaattcat accacatgtc acagccagta caaagctggg atgtttaagt aagatttgaa 39420
agccaaggga agacaccagt aaaataaaca aagggtaata tgagactaag gtccccagct 39480
cataatttta tttatttatt tgttttgacc ccaacatgca ttctacaatg gagaagagct 39540
cacaagtcat aacgtatttc agtgtgtgga caagtacaag agttgttgta acgttcagaa 39600
gagttcatcc taatctccac ccaaggggtt ccctgaaagt tatctatact cctcaaagtg 39660
tagacttgag agaggaaagc ccaatgagag aggaggagtt cttccttttt ctctgttctg 39720
aacccaacct ctgtgtgctc aaagtcatat gaacaaagat gttcatcaca actttgtctt 39780
agtaaaacac cagaatcccc ctcccagatg accgttcata caaagcaagc tagcttgtgg 39840
catatcccca ctataatggc atagatatat atttgacatg gaaatatgtg caagccatat 39900
tgtggaaaca atgaaacagg tttttttagt ccatttgtgt tgctgtaaag gaatacctga 39960
ggctgggcaa tttatcaaga aaagaggttt atttggctca tggttctgca gaccatacag 40020

gaagcatgac accagcatct gcatctgatg agggcctcag actgcttctg ttcgtggtgg 40080
aaggggaaga ggcgctggca tatgcagagg tcactgatga gggagagaga gagagagaga 40140
aagcaagaga ttaggggttg gtaggtgcca ggctgttttc aacagccagc tctagaagga 40200
actaatggag tgacaactca tcatggagag gacagcacca agacattcat gagggatctg 40260
cccccaccac ccaagcacct ctttttagga cccacctcca acattgggga tcaaacttcc 40320
atttgaggtt tggagggcca aatatccaaa ctatagcaag gttataaaat agtatctata 40380
gaaggggctc tttttctctt cgctgaacca tttgttagta taagcatggg ctctaagggc 40440
agaccacctg gatttaaatc ccagctccta catcccctaa ttctactcac tgtcccctca 40500
atgcaattac tctccaaatc caaatgccac tcaagctttt cctcaaccat ggaacacagt 40560
attctcccct ctatatctac ctatggaaat cctagtcttt ttcaagatca agctctccat 40620
cgcatctcct taaagcttgc ctttgcttct cctctatcca ggagatccac ccttcagaga 40680
attcctctgg tactcactgt ctaaggagct gctttgaaaa gatcttttcc actatattag 40740
tccattcagg ctgctatagc aaagttctat agattggata tcttataaac aacagaaatg 40800
tattcctcat ggttctggag actggaaagt ccaagaacaa ggtgcaggcc aacttggtgt 40860
ctgacgaggg cccactttct ggttcataga tggtgccttc ttgctgtgcc ctcacctagt 40920
aaatgaggca aaagagcttt ctggagcctc ttttatgagg gtactgatct cattcatgag 40980
ggttccactc ttgtaaccta atcacctctc aaaggcccca cctcccaata ttatgccatc 41040
cccttgggta ttaggtttca atatatgaat tttgagggac acaaacattc actctgtgtc 41100
acctaacctg tatttgttga ctcacctttt atgtataaat accttatttt cctaaataag 41160
tttacacttt tgaggccagg aaacatacag atggtaagtg ctttgtgttt ctcaattaat 41220
ttcaggcccc tgcaacacat caaaccacag tcgcatagtt ataataccca agaactttgc 41280
tataatgctg tccagcatca cccaggatag gtactctcat attctgttgg taaggaggta 41340
cagccctttg gacagtaatt tgtctgcagc tctcaaaatt ttaaatatct atacattttg 41400
acctggtgac atggtttgaa tgtttgtccc tccaaatctc atgttgaaat gtaatcccca 41460
gtgttggagg tggagccagt aggaggtatt tggatcatgg gggcggattc ctcatcaatg 41520
gcttggtgac gagtgacttc atctgagatc tggttgttta aaaatgtatg gcacctccct 41580
aactccctct cttgctcctg ctctccccac acgacccatc tgctcaccct ttgccttctg 41640
ccatgattgt aaggttcctg agcccctcac tagaagcggg tgctggagcc atgcttgtac 41700
agcctgtaga actgtgagcc aattaaacct cttttcttta taaattaccc agtctcaggt 41760
attcctttgc agcaacacaa gaatggccta acatacctgg caatttctag aagtgtgtcc 41820
caaagaaata ccctcaaaac tgtctaaaaa tatgtatgaa taggaatatt tgcagcattg 41880
tttataatca tgaaaaattg gaaacaacct aaatgctcaa cattaggagg ttgaacaact 41940
tactgcaatg aaatactagg taacgtttaa gaagactaag tccaaatata ctgacatgga 42000
aagatgccca agatatacca tatattatta aaaggaaatt gccaaacgtt attaaatgac 42060

ttatttttta aaacatgcat atagatgtat ttgcatgcat agaaaaagta tctgtttcac 42120
accaaattgt caatagaagg tgtccctagg aataggatag gaagtgggga aatgttctcg 42180
ttttacttta tgcacttcca gaacatttga atttttttca aattttaaat ttataattct 42240
ttaaacatag attcaccaaa agagataaac acgatggtcc attatacatt catcaagaag 42300
tttaattact agcatgccca cttcattatc aagcagttgg ggcacaggca tgggcacaag 42360
ggcctcggca ggctgcgctg tggcttaagc ctcagcagag tttcagaccc tgcttttttt 42420
ccctgaggat ggcatacaag atgttcaatt agccatagca gttgaggtca gggagggata 42480
aaatgggcaa gggagccaag agggtgctgg cccagagcaa ggtcggggtg aagccacaca 42540
gactccaccc attcaaaact gtcctgttgg gccaggcacg gtggctcaca cctgtaatcc 42600
cagcactttg ggaggctgag gtggatcact tgaggtcagg agttcgagac cagcctggcc 42660
aacatagtga aaccccatct ctactaaaaa tacaaaaatt agccaggtgt ggtggcaggt 42720
gcctgtaatc ccagctagtg gggaggctga ggcaggcgaa tcacttgagc ccaggaggca 42780
gaggttgcag tgagccaaga ttgcaccact gcactgcagc ctgggcaaca gagtgagact 42840
ctgactcaga aaacaaaaca acaacaacaa caacaaaaga actgaccccc ccaacccaac 42900
cttccctccg gattactctc caatccctct ggcctctcaa atcatattta atgcccttct 42960
agttctcaca gtttagccct aaattatttt cattgtctct ttccaatcag tcgtaagtgg 43020
aatgcatgat agcattttga agtcaatgag aggattctag tccttccctc tccctcaggt 43080
cctttctgat ccaatagtct gaaagtttga atatgtattt tgcttatgtt aaggctttaa 43140
gacagaacaa aatgcacata tctctctagg aggtcccatc ttattctgtt ctcagacaat 43200
gacaccttac aagaaatgga ggattgggga aagagttgga tctggggttg gtgggaagaa 43260
ggaagtggat ggcaaagccc agactgaggg tgggtaggaa gtgtggagga tgtaatgaaa 43320
ctgggcacac tagaggggcc ccgtctacct cacttagctc gcttagcccg gtcacctgag 43380
gaccatttta aaaatgcaga tgcctaagcc ctgcttccaa tattctaatt tgattggcct 43440
agcatgaggc ccaaatatct gtcatttgaa agccttccag gtaatgcaga catgctgtca 43500
ggtgtgacat gactaagtca gagggagacg tttgtactca cagactggga agacagggga 43560
cgcacagaaa ggatgcaagt cagcccaatg tctgacccca cagggagggg acctgctctg 43620
aatggctggg gatctgacac aggctctcgg ctcctggagc acttaccttt cctaaccctg 43680
gaggatgcag tcttggggga gaaagagcaa gtcctacaag cagggaaatt aggacaaacc 43740
ggcccctcct cagctgtccc actgcgctgc ccagggaaag ctcaggctgc tggtgccttt 43800
gtacaacttc tgagccattt cctggccttt gaaatgagcc ctttccatgg gcagaacaaa 43860
gtgaagagtt aaggaaaatt tccacataaa acagactcca aatttagcta ttaaggaacc 43920
agactcccaa ggcagccatt agcaaagcta agattcactg gctatccgca cagaacttca 43980
ttttgtcttt tgggaatttg ggatgaattc ggattagaaa caaacatttt aaataacatc 44040
ctccaaaaat ctggttcagc aattagcagc ttcatttaat tacaggggaa ggagaggaca 44100
gagagaaaga gggagagaga aaaaaaagag gggcattgat ggctgaaggg tttttctttt 44160

ttcctctctt gcaaataatt cctttaacca ttccaacacc aaatagaaag aattttaaaa 44220
atatatttac agataagctg tcggcctctt ccccgtgaag aagtaaatca gatccatgag 44280
gactgcaagc acatgggact cctcgttggc agaggcagca ttttttctca ggtaagagaa 44340
agactggttt ttacctatcg ctctaggtag gagtctctct gatttcctaa tgctggtcac 44400
agtttcttca cagctgtgaa gatgtcctaa tgctggttgc agtttttaca caactgtgcc 44460
gaggtaccca gccacatgtc atctagggat gaagctcggg tacaaaaaaa aaaaaggcgg 44520
agacagtgca gctctcaggc cactaggggg cgctgagtga aaatggtttg gttgtcgcgg 44580
ctctttgggg taaagataga ggcagacaga ggatggtcaa agcggacaac taacagtatg 44640
gatgataaac atcacagtca tgcagtaaat aatctatctc ctgtataatt aattctaacc 44700
cctgtgaaag caattctggc cgttcttagc tcagcccagg gctctgactt ggtgaccctg 44760
ctaagcctta gtggcctctg tggctgggag caacacgaca gcacctgcag gcccgggtga 44820
cgcctgtcgg agagatggag gaccacctgg gcatagcccc cattgccaaa ctcaaaatca 44880
gccagagcca gtgtccttca cccttaggct gggagattca gaggtcgagc cctccccact 44940
cccttccttg aaaagccatg cccagctcca cagtcaggac cctggcttgc cctgcacaca 45000
ctgcccgaga ggctgcccct cacagcaaca gcctctcccc agggatgggc tacagggtgg 45060
agacttaaga gagaaggtaa cttctgctcc tccctaattt gtcctgtcct ttgttaactt 45120
tctttcccat tttaaacatt cctcatcctt cccagtttcc ctgctccatc cctgcctctc 45180
acaacttcag tggatactga aatatctgct tcttgcctca tacttctcag ataaaattga 45240
aactatcagg caaaacctct atataaactc atcggtcccc gcatttattc tttcctcccg 45300
tgttagtccc cccccccagc ctgctatcac aaagcgccat agcctgggtg gcttatacat 45360
catagaagtt catttctcat agttctggag gcagagaagt ccaagatcaa ggtgccagca 45420
agttcagtgt ctggtgaggg cgctctttct aagcggccat ctttctgctg tgacctcaca 45480
ttgcagaagg ggcaagagag ctccctgggc cttcatgggc tttgccctcc tgagctaatc 45540
acttcccaaa ggctccactt cacaagacca tcaccttggg agttagggtt ccaacatagg 45600
aattcaaggg gtagaggcac aaatgtccag atcatagcac ttccttccct gaaggttcca 45660
gaaatgaggc atttctcctt ttcaaatcta gatcttccat ctgtgtgctt gtttctccaa 45720
gctccctcca ccttcactca aggaaacccc catgttttct tgcatcttca atttttcccc 45780
aacttcccct tcccctttaa acaaaacaga cttaaagttc tcccagccca aacaacaaag 45840
ccagaatgag cacttttgct gggatgtgct ctgtgatttt ttttctagtc caggtcataa 45900
atatttttct gtacaaacca gataacataa tttaatgatt atgaatcgta agacatgcaa 45960
tttataaaga catgagatac ttgtgtactt gcttagctct tcctttgtta tttggcaaaa 46020
tttgttatgt gctaagcaac aggtgtcatt ctggaaactg aaactattag gttgctgtaa 46080
aagtaattgc agtttttgtg atttttttta attgcaaaaa ctgcaattac ttttgcacca 46140
acctaatacg gtgataaata agatctggtc cttccttcgt ggagctggtg ggagagaaag 46200

gcaaatacat agaaaattac aatataatgt agtaagctca tctataaata ttttttaaaa 46260
gatattcaag ggggaaagag aagggacttc taggccaggc tttctgggta gcagaatctt 46320
aaataatgag ataaacaacc aaactgtgca tgaaatgtgg cagagtacaa agaactaatt 46380
ttgcacacca catcacacca cacagatgtg gctggtgcaa aggcttgagt agtcagtgga 46440
gctggtatga agcccttcct gcttgtcagg cactgtccta acactttgca tgaactgact 46500
catttaatgc tcccaacaag cctataaaat aagtactatt actatcctta ttttacagat 46560
aaaggaaaag acacacagag aggttaagta atgacagaac caggatccaa actcaggtag 46620
tctgatttca gaatctgtcc tctgaggaag acaaatctaa ggggacaaga ctggagacag 46680
ggggtccctc tgggagccaa ggaggctgtt tgaacctgag ctgtggtagt agagatagag 46740
gagatattct gaggataatc ctggaaaatg cacagactgt ggatatgaag aaccagagag 46800
agtcgaggaa gaagctctca tgttggtttg gagtgggcaa gaatgagtcc agctttgagc 46860
atgctccatt caaggtgttc ataaacatct cactctatct acagatgctt cttgacttat 46920
agtggggtta cctcctgata aacccctcct aaattgagaa tattttaagt caaaaatgca 46980
gttaatacac ctaaccaact gtacatcata gcttagccag cataccttaa atgtgctcag 47040
aacacttgca ttagcctaca gttgagcaaa gtcatgtgac aaagcctatt ttataataaa 47100
gtgttgagta tctcatgtaa tttattgact actgtactga aactgaaaaa cagaaaggtt 47160
gtatagatac ttgatgtaca atttctacta atgtgtatca ctttcccatc attgtaaagt 47220
caaaaatatt ctgtcaggga ccatctgtag aagtctgtag ctcggaggaa atgactgaag 47280
atcatgtttt ataggtcatc agcatttaca cagtgagatg aggaaatctc ccaggaagtc 47340
taatgtctag atgagaagct cagtgagatg aagatagaaa cctgcacaac actgactttt 47400
aacgggcagg agaggccggg cacggtggct cacgcctgtg atcccagcac tttgggaggc 47460
cgaagcggca gatcacttga ggttaggagt ttgagaccag cctagccaac atagtgaagc 47520
cctgtctcta ccaaaaatat aaaaaattag ctgagtgtgg tggcacacac ctgtaatccc 47580
tgctactcag gaggctgagg caggagaatt gtgtgaacct gggaggcgga ggttgcagtg 47640
agccgagatt gtgccactgc actccagcct gagcgacaga gtgagactct gtctcaaata 47700
aaataaaaat aataacagta ataataataa aaggcaggag aaggaaaaag ctgcccaggt 47760
agaggcagaa agcgtggaca ggtatgaaga gaataagaag agtgtgaagt catggaagaa 47820
agagacagga gaggttcagg aagtggggga attccaaaga gtcaagtgca gagaaggcca 47880
gagagaatga gggctggaag atgaccagtg gattccacac ttgcaggtaa ctttagtaga 47940
gtggttgatg aggcagaagt cagactgcaa ccaattgaga tgtgaatggg ggaagggcaa 48000
agtaataaat aatggccaat aagcaccaac cagtcttttt agtgatgtgg ctaaaagcag 48060
gaagaaagca tttgcgaggt agccataggg gacaaggggg tcaacaaaga tttgtatttg 48120
gtgtggtcta gattggggtt atgagagctt taagcctgct tgtttatagg ctaaggggaa 48180
gttgaagggg cgtttcctgg agtgaaggag atgggagaat ccagggtcaa gggcataggt 48240
ggacaatttg gagttgaaca gcaaatacac cttgttctct gagcctttag gaaagtttat 48300

aaaaatggat gtgagagcaa acaagtttgt cttgcgtggc agcttcaatt ttctgagaag 48360

tatgaggcat gattgtctat gaaatagagg gagaggcgca ggagttgagt aaggaaattg 48420

agaggaatga taaaggttta gattgagaaa ggacgctgac aatgaacaag aaaagttggg 48480

gtgacccaca gaagttggag accctgaata tctagcggca tcactcagcc tagctgtgtg 48540

atttttctct aactcttctc agccatctag attagaaaaa agccattcaa taaaatattt 48600

cattagcgct gggattggct gtggtgtggg tgcagcagaa aaagggtaga aatgcaagca 48660

aaatgagggg caagtgtgag cctacttcat tcaactctgc gttctgagct gggtagaaag 48720

gaaggaggag acctgagtgg agcaatagac ctgaaaacaa tgggtgaatg aggattctga 48780

agtcttcatg aaaaggaaag ctgtagtggg aatcagaaat tgagttgttc ctggtgaaga 48840

ggaggctcat ggcattggcc atgggtatga atggttgcag aagggtggac ataaagattc 48900

tgggagttgt ggcaataagg gcagtgagaa gtgagtttgc tgggttgcac atatggacat 48960

ggaagtcacc ctgggcagaa atggagtata gcgaggggcc taagacttca gtgaaggtgt 49020

agaatgacca ggaggtaaat gtatgacaaa aaggaaaagg ggcagagcca aattgtacaa 49080

gggtttgtat ggagactgga gggtgaatgg cctggattca gcattgggga tggaggagga 49140

accaaactga gagaaacatg agaaacatcc taaacacaag gttttggatt tttagtttag 49200

tgcatttcct caaagtcata tttgattctc tgatgatttg ttttcttatt ttttggtgtc 49260

tcctcagaca tttcgcattg cgccctcaat gtgcatcact aaaccagaag ttgattttgc 49320

agtagaagta tttcgttctg ccttaaccca acacatggaa agaagagcta agtaacattg 49380

tcagaaataa ataaaaccac aagtctcaag aatttgccac gtatgttcaa gggtgaattt 49440

gaagaatttc agaaccactg gtatccagag aaagcctgca gctctccaca ggagctgtaa 49500

aagtcatggt tgactgccta ccaaccatat ttgttagcag agcccctctt atcttgagaa 49560

ctccattctt cagggaaagg atctccctag ctcagagaat aaatcctaat tagtttatgt 49620

taggtatggt aatttgattc ccctttgcag tgattggttt atgcatgaat atgtgatgta 49680

tttttgtcca gtgaatcttg aagaaaaatc ttttggtgga ggtgccttca gggaaagttt 49740

tcttcaccct cactcttcag ttcaagaaga gatgtcttct tgttgcgctg agaacaccat 49800

atgttcatga cgagattcct ggcaccatgt cagccggctt gtagtcatga ggacaaccct 49860

ttttggtgag gttggaagat ggatggaagc caagtgctta gtgatgtcaa agaagcactc 49920

acttaagcat tcctggagcc accctacctc agggcctctt gatatttgag gtaataaatt 49980

cattgttctg tat 49993

<210> 13
<211> 2165
<212> DNA
<213> Homo sapiens

<400> 13
aaatgactct aatctggaga catttgctga gacccttgtg cctggtcact tccgctccca 60

ggatccttga gatgcatcct ttcctgagcc taggtacttc ccggacatca gtaaccaagc 120

tcagtcttca tacaaagccc agaatgcctc catgtgactt catgcctgaa agataccagt 180
cccttggcta caaccgtgtc ctggaaatcc acaaggaaca tctttctcct gtggtgacgg 240
catatttcca gaaacccctg ctgctccacc aggggcacat ggagtggctc tttgatgctg 300
aaggaagcag atacctggat ttcttttccg ggattgttac tgtcagtgtt ggccattgcc 360
acccaaaggt gaatgcagtg gcacaaaagc agctcggccg cctgtggcat acaagcaccg 420
tcttcttcca ccctccaatg catgaatatg cagagaagct tgccgcactt cttcctgagc 480
ctcttaaggt cattttcttg gtgaacagtg gctcagaagc caatgagctg gccatgctga 540
tggccagggc gcactcaaac aacatagaca tcatttcttt cagaggagcc taccatggat 600
gcagtcctta cacacttggc ttgacaaacg tagggaccta caagatggaa ctccctggtg 660
ggacaggttg ccaaccaaca atgtgtccag atgtttttcg tggcccttgg ggaggaagcc 720
actgtcgaga ttctccagtg caaacaatca ggaagtgcag ctgtgcacca gactgctgcc 780
aagctaaaga tcagtatatt gagcaattca aagatacgct gagcacatct gtggccaagt 840
caattgctgg atttttcgca gaacctattc aaggtgtgaa tggagttgtc cagtacccaa 900
aggggtttct aaaggaagcc tttgagctgg tgcgagcaag gggaggcgtg tgcattgcag 960
atgaagtgca gacaggattt ggaaggttgg gctctcactt ctggggcttc caaacccacg 1020
atgtcctgcc tgacattgtc accatggcta aagggattgg gaatggcttt cccatggcag 1080
cagtcataac cactccagag attgccaaat ctttggcgaa atgcctgcag cacttcaaca 1140
cctttggagg gaaccccatg gcctgtgcca ttggatctgc tgtgcttgag gtgattaaag 1200
aagaaaatct acaggaaaac agtcaagaag ttgggaccta catgttacta aagtttgcta 1260
agctgcggga tgaatttgaa attgttggag acgtccgagg caaaggtctc atgataggca 1320
tagaaatggt gcaggataag ataagctgtc ggcctcttcc ccgtgaagaa gtaaatcaga 1380
tccatgagga ctgcaagcac atgggactcc tcgttggcag aggcagcatt ttttctcaga 1440
catttcgcat tgcgccctca atgtgcatca ctaaaccaga agttgatttt gcagtagaag 1500
tatttcgttc tgccttaacc caacacatgg aaagaagagc taagtaacat tgtcagaaat 1560
aaataaaacc acaagtctca agaatttgcc acgtatgttc aagggtgaat ttgaagaatt 1620
tcagaaccac tggtatccag agaaagcctg cagctctcca caggagctgt aaaagtcatg 1680
gttgactgcc taccaaccat atttgttagc agagcccctc ttatcttgag aactccattc 1740
ttcagggaaa ggatctccct agctcagaga ataaatccta attagtttat gttaggtatg 1800
gtaatttgat tcccctttgc agtgattggt ttatgcatga atatgtgatg tatttttgtc 1860
cagtgaatct tgaagaaaaa tcttttggtg gaggtgcctt cagggaaagt tttcttcacc 1920
ctcactcttc agttcaagaa gagatgtctt cttgttgcgc tgagaacacc atatgttcat 1980
gacgagattc ctggcaccat gtcagccggc ttgtagtcat gaggacaacc ctttttggtg 2040
aggttggaag atggatggaa gccaagtgct tagtgatgtc aaagaagcac tcacttaagc 2100
attcctggag ccaccctacc tcagggcctc ttgatatttg aggtaataaa ttcattgttc 2160

tgtat                                                                    2165

<210>  14
<211>  514
<212>  PRT
<213>  Homo sapiens

<400>  14

Met Thr Leu Ile Trp Arg His Leu Leu Arg Pro Leu Cys Leu Val Thr
1               5                   10                  15

Ser Ala Pro Arg Ile Leu Glu Met His Pro Phe Leu Ser Leu Gly Thr
            20                  25                  30

Ser Arg Thr Ser Val Thr Lys Leu Ser Leu His Thr Lys Pro Arg Met
        35                  40                  45

Pro Pro Cys Asp Phe Met Pro Glu Arg Tyr Gln Ser Leu Gly Tyr Asn
    50                  55                  60

Arg Val Leu Glu Ile His Lys Glu His Leu Ser Pro Val Val Thr Ala
65                  70                  75                  80

Tyr Phe Gln Lys Pro Leu Leu Leu His Gln Gly His Met Glu Trp Leu
                85                  90                  95

Phe Asp Ala Glu Gly Ser Arg Tyr Leu Asp Phe Phe Ser Gly Ile Val
            100                 105                 110

Thr Val Ser Val Gly His Cys His Pro Lys Val Asn Ala Val Ala Gln
        115                 120                 125

Lys Gln Leu Gly Arg Leu Trp His Thr Ser Thr Val Phe Phe His Pro
    130                 135                 140

Pro Met His Glu Tyr Ala Glu Lys Leu Ala Ala Leu Leu Pro Glu Pro
145                 150                 155                 160

Leu Lys Val Ile Phe Leu Val Asn Ser Gly Ser Glu Ala Asn Glu Leu
                165                 170                 175

Ala Met Leu Met Ala Arg Ala His Ser Asn Asn Ile Asp Ile Ile Ser
            180                 185                 190

Phe Arg Gly Ala Tyr His Gly Cys Ser Pro Tyr Thr Leu Gly Leu Thr
        195                 200                 205

Asn Val Gly Thr Tyr Lys Met Glu Leu Pro Gly Gly Thr Gly Cys Gln
    210                 215                 220

Pro Thr Met Cys Pro Asp Val Phe Arg Gly Pro Trp Gly Gly Ser His
225                 230                 235                 240

Cys Arg Asp Ser Pro Val Gln Thr Ile Arg Lys Cys Ser Cys Ala Pro
                245                 250                 255

Asp Cys Cys Gln Ala Lys Asp Gln Tyr Ile Glu Gln Phe Lys Asp Thr
            260                 265                 270

Leu Ser Thr Ser Val Ala Lys Ser Ile Ala Gly Phe Phe Ala Glu Pro
        275                 280                 285

Ile Gln Gly Val Asn Gly Val Val Gln Tyr Pro Lys Gly Phe Leu Lys
    290                 295                 300

Glu Ala Phe Glu Leu Val Arg Ala Arg Gly Gly Val Cys Ile Ala Asp
305                 310                 315                 320

Glu Val Gln Thr Gly Phe Gly Arg Leu Gly Ser His Phe Trp Gly Phe
                325                 330                 335

Gln Thr His Asp Val Leu Pro Asp Ile Val Thr Met Ala Lys Gly Ile
            340                 345                 350

Gly Asn Gly Phe Pro Met Ala Ala Val Ile Thr Thr Pro Glu Ile Ala
        355                 360                 365

Lys Ser Leu Ala Lys Cys Leu Gln His Phe Asn Thr Phe Gly Gly Asn
    370                 375                 380

Pro Met Ala Cys Ala Ile Gly Ser Ala Val Leu Glu Val Ile Lys Glu
385                 390                 395                 400

Glu Asn Leu Gln Glu Asn Ser Gln Glu Val Gly Thr Tyr Met Leu Leu
                405                 410                 415

Lys Phe Ala Lys Leu Arg Asp Glu Phe Glu Ile Val Gly Asp Val Arg
            420                 425                 430

Gly Lys Gly Leu Met Ile Gly Ile Glu Met Val Gln Asp Lys Ile Ser
        435                 440                 445

Cys Arg Pro Leu Pro Arg Glu Glu Val Asn Gln Ile His Glu Asp Cys
    450                 455                 460

Lys His Met Gly Leu Leu Val Gly Arg Gly Ser Ile Phe Ser Gln Thr
465                 470                 475                 480

Phe Arg Ile Ala Pro Ser Met Cys Ile Thr Lys Pro Glu Val Asp Phe
                485                 490                 495

Ala Val Glu Val Phe Arg Ser Ala Leu Thr Gln His Met Glu Arg Arg
            500                 505                 510

Ala Lys

<210> 15
<211> 24
<212> DNA
<213> Artificial

<220>
<223> oligonucleotide primer

<400> 15
gcggaattcc atgactctaa tctg 24

<210> 16
<211> 21
<212> DNA
<213> Artificial

<220>
<223> oligonucleotide primer

<400> 16
atggtgcttg tatgccacag g 21

<210> 17
<211> 22
<212> DNA
<213> Artificial

<220>
<223> oligonucleotide primer

<400> 17
cttcttccac cctccaatgc at 22

<210> 18
<211> 23
<212> DNA
<213> Artificial

<220>
<223> oligonucleotide primer

<400> 18
gagctcgagt cgagttactt agc 23

## Description

The present invention generally relates to methods for determining the mortality risk in a subject. In one aspect, the invention relates to a method for determining/predicting the all-cause mortality risk of a test subject based on the detection of pathologically decreased expression levels of the gene encoding the alanine-glyoxylate-aminotransferase 2 (AGXT2) and/or pathologically decreased activity levels of the AGXT2 enzyme. In a further aspect, the invention relates to a method for determining/predicting the all-cause mortality risk of a test subject based on the detection of genetic alterations which are associated with decreased expression levels and/or activity levels of the AGXT2 enzyme. Finally, the invention also relates to methods of screening for compounds which are effective in increasing the expression of the AGXT2 gene and/or the activity of the AGXT2 enzyme.

### BACKGROUND OF THE INVENTION

Acute ischemic stroke is one of the major public health problems causing high rates of mortality and morbidity in cardiovascular patients. Consequently, considerable effort has been put into the evaluation of factors that may provide predictive information on morbidity and mortality after the acute event. Besides the traditional risk factors (advanced age, hyperten-sion, atrial fibrillation, diabetes mellitus, and smoking), factors involved in hemostasis were found to predict mortality after acute stroke, including von Willebrand Factor (vWF) and β-thromboglobulin (βTG) (Carter et al. (2007), Stroke, 38(6): 1873-80) which reflect the activation of endothelial cells and platelets. Besides these well known risks, identification of vulnerable patients is still a challenge for modem medicine. Thus, also the identification of bio-markers which are related to stroke outcome is highly desirable.

Recent data has suggested that high plasma concentrations of symmetric dimethylarginine (SDMA) may be associated with cardiovascular and all-cause mortality in patients subjected to coronary angiography or hemodialysis (Schulze et al. (2010), Artherosclerosis, 208:518-523). SDMA is an endogenous analogue of the amino acid L-arginine which contains two methyl groups. Endogenously formed methylated amino acids may impair metabolism and transport of their unmethylated form and thereby exert adverse effects on human health. For example, it was shown that SDMA interferes with the L-arginine/nitric oxide (NO) pathway: SDMA inhibits cellular L-arginine uptake by inhibiting the accordant transport system (Closs et al. (1997), Nitric Oxide, 1(1):65-73). SDMA is formed by post-translational protein di-methylation. After hydrolytic protein degradation of dimethylated proteins, SDMA is released and may be carried from cytoplasm to the extracellular space. SDMA was reported to be eliminated almost exclusively through the kidneys into the urine. Accordingly, SDMA has been suggested as a novel marker of renal function impairment (Kielstein et al. (2006), Nephrol Dial Transplant 21:2446-51).

It was suggested that elevated plasma levels of SDMA may be associated with cardiovascular disease events (Kiechl et al. (2009), Artherosclerosis, 205:261-5). SMDA was also significantly associated with the presence of cardio-embolic stroke (Wanby et al. (2006), Artherosclerosis, 185:271-7). Moreover, it was recently shown that elevated plasma levels of SDMA are predictive of adverse clinical outcome and all-cause mortality after ischemic stroke (Schulze et al. (2009), Artherosclerosis, 208:518-523).

Against this background, it has been found by the present inventors that the SDMA concentration in the plasma of a subject is related to survival of said subject after acute ischemic stroke. Further, it was found in a genome-wide association study that the formation of SDMA is directly associated with the activity of AGXT2.

As described herein below, the inventors found that the catalytic activity of the AGXT2 enzyme is required for the degradation of SDMA in humans. Moreover, SDMA plasma concentrations were monitored in 394 patients after acute ischemic stroke during a median follow-up of 7.4 years, and it was found that SMDA was associated with survival (Hazard ratio (HR) 3.70 (95% CI 2.46-5.57)) (Schulze et al. (2010) see above). SDMA was a significant predica-tor of mortality in multivariate analyses indicating that SDMA is an independent risk for death from any cause in these patients.

This insight provides the possibility of measuring the SDMA concentration in a subject or, alternatively, the expression level of the AGXT2 gene and/or the activity of the AGXT2 enzyme to assess the individual mortality risk of said subject. Based on the insights provided by the present invention, diagnostic tests can be developed which rely on the detection of alterations in the genome of a test subject that are associated with a decreased expression of the AGXT2 gene and/or a decreased or abolished activity of the AGXT2 enzyme which is in turn an indication of an increased mortality risk.

In the present invention, a genome-wide association (GWA) study in a population-based study was conducted. In this manner, a number of single nucleotide polymorphisms (SNPs) were identified in the gene encoding the alanine-glyoxylate-aminotransferase 2 (AGXT2) in humans. In addition, it was shown that transfection of the human AGXT2 gene into HEK cells which do not show AGXT2 mRNA expression results in the capacity of degrading SDMA in a specific AGXT2 activity assay that uses stable isotope-labelled SDMA as substrate. By contrast, cells which express AGXT2 with a mutation as found in the GWA show a dramatically impaired ability to degrade SDMA.

### BRIEF DESCRIPTION OF THE FIGURES

Figure 1 depicts a plot showing the genetic association of SDMA plasma concentration and AGXT2 gene on chromosome 5.
Figure 2 shows the SDMA plasma levels of patients suffering from ischemic stroke stratified for different AGXT2 genotypes concerning the SNP rs37369 (SEQ ID NO:2).
Figure 3 shows the degradation of NADH in ng/min/g protein, detected by measuring the absorbance at 340 nm, in mouse kidney tissue lysates which are incubated with L-alanine or SDMA as alternate AGXT2 substrates. The decrease in NADH concentration correlates with the increase in pyruvate concentration and is a measure of AGXT2 activity.
Figure 4 shows the degradation of d6-SDMA by HEK293 cells in culture. Cells were transfected with an expression vector containing the sequence of wildtype AGXT2, of an AGXT2 containing the SNP rs37369 (SEQ ID NO:2 in Table 1), or empty control vector. The reduction of d6-SDMA (initial concentration, 0.5 µmol/l) in the presence of 1 mmol/l pyruvate, 1 mmol/l glyoxylate, and 0.3 mmol/l pyridoxalphosphate in PBS (pH 9.0) correlates to the activity of AGXT2.

### DISCLOSURE OF THE INVENTION

The invention relates to both diagnostic and therapeutic aspects which will be described in more detail below. Specifically, the present invention provides means and methods for determining the all-cause mortality risk in a subject by assessing the expression level of the AGXT2 gene, the activity of the AGXT2 enzyme, and/or by detecting genomic alterations which are associated with a decreased expression of the AGXT2 gene and/or a decreased activity of the AGXT2 enzyme.

This means that the methods of the invention enable the prediction of an individual's general risk to die from any cause. In other words, the reason for death is not specifically limited to particular pathological conditions or diseases. Instead the all-cause mortality risk reflects the risk to die from any possible cause, wherein the fatal outcome of pathological conditions and diseases of course contribute to mortality.

According to a particular preferred embodiment of the invention, the all-cause mortality risk referred to in connection with the methods disclosed herein is the all-cause mortality risk after having experienced a cardiovascular event, i.e. the risk to die for any cause after the onset of a cardiovascular condition. A cardiovascular condition in the sense of the present invention is meant to refer to any disease affecting the cardiovascular system, including hypertension and complications thereof, ischemia and complications thereof, acute coronary syndrome (ACS), coronary artery disease (CAD), myocardial infarction, heart failure, angina, cardiac hypertrophy, arteriosclerosis, peripheral artery disease, vasospastic disease, myocarditis, pericarditis, endocarditis, stroke, such as acute ischemic stroke and/or pulmonary embolism and/or transient ischemic attack (TIA), diabetes mellitus, atrial fibrillation, lacunar infarction (LACI), anterior circulation infarction (TACI). The cardiovascular condition may still be present at the time of subjecting the subject to one of the methods of the present invention, or it may be cured with no detectable clinical signs of the condition or diseases remaining. In an even more preferred embodiment, the all-cause mortality referred to herein is the all-cause mortality after having experienced a stroke.

The test subject referred to in the different methods described herein will normally be a mammal, preferably a human. Generally, the subject can be of any age. Where the test subject is a human, such subject will be older than 20 years, more preferably older than 30 years, 35 years, 40 years, 45 years, 50 years, 55 years, 60 years or 65 years. For example, the human subject will be between 30 and 65 years old, e.g. between 35-60 years, 40-60 years, 45-65 years, 50-65 years, and most preferably between 55-65 years. Preferably, the test subject is currently suffering from or has previously experienced a cardiovascular event, such as a stroke.

The reference subject referred to by the methods of the invention will be of the same species as the test subject. Thus, where the test subject is a human, the reference subject will also be a human. The reference subject will preferably be a subject that is not afflicted with any health problem or condition which is known to shorten life expectancy. Preferably, the reference subject will exhibit a SDMA concentration in the plasma which is typical of the general population of normal, disease-free subjects. The mean plasma concentration of SDMA in humans is in the range of 0.225 to 0.533 µmol/L.

In an alternative embodiment, the methods of the invention may also use a reference subject which suffers from a disease or condition which is known to affect life expectancy, e.g. a cardiovascular disease, such as a stroke. In this case, the SDMA concentration of said reference subject in the blood or serum is higher than that measured in the general population of normal, disease-free subjects. If the AGXT2 expression levels and/or the AGXT2 enzyme activity levels essentially correspond to the levels measured in the latter reference subject, this indicates that the mortality risk in the test subject is increased.

Further, the invention provides methods of screening for novel compounds effective in increasing the expression of the AGXT2 gene and/or the activity of the AGXT2 enzyme. These compounds can be used to decrease the mortality risk of a subject, preferably after a cardiovascular event, such as a stroke.

### Diagnostic methods targeting expression levels

In a first aspect, the present invention relates to a method for determining/predicting the all-cause mortality risk of a test subject, comprising
(a) detecting in a sample from said test subject the expression level of the alanine-glyoxylate-aminotransferase 2 (AGXT2) gene;
(b) comparing the expression level of step (a) to the expression level of said gene in a reference subject;
wherein detection of an expression level of the AGXT2 gene which is significantly decreased in the test subject relative to the expression level in the reference subject indicates that the test subject has an increased mortality risk.

The above method is carried out with a biological sample that has been obtained from the test subject. A variety of different sample types may be used, provided that the sample contains cells which would express the AGXT2 gene in a normal, healthy subject, i.e. a subject having normal SMDA concentrations in the serum. The distribution of the AGXT2 mRNA in humans was determined by RT-PCR (data not shown). The strongest mRNA expression of AGXT2 was found in liver and kidney followed by placenta, heart, pancreas, skeletal muscle and lung. There was no amplification of AGXT2 in the brain. Preferably, the sample in which the expression level of the AGXT2 gene is detected is a liver, kidney or blood sample, more preferably a blood plasma sample. The sample can be a tissue sample which has been obtained from the test subject, e.g., by core needle biopsy, fine needle aspiration, and the like. Before being used in the methods of the invention, the sample can be processed to render the sample eligible for expression analysis, e.g., by cell lysis and subsequent RNA purification. In a particularly preferred embodiment, the biological sample obtained from the subject is a blood sample, and the expression level of the AGXT2 gene is measured, e.g., based on RNA (in particular mRNA) extracted from white blood cells, such as lymphocytes, monocytes and/or neutrophils.

For evaluating the mortality risk of the subject to be tested, the extent of expression of the gene encoding the AGXT2 enzyme is analyzed. The complete human AGXT2 gene, including intron sequences, is disclosed as SEQ ID NO:12 herein. The coding sequence of the human AGXT2 gene, i.e. the sequence obtained after splicing of the mRNA has occurred is depicted in SEQ ID NO:13. Preferably, the methods of the invention which are directed to the detection of expression levels make use of the mRNA sequence corresponding to the human DNA sequence depicted in SEQ ID NO:13. It will be appreciated that the polynucleotide sequence of the human AGXT2 gene can exhibit a number of sequence deviations resulting, e.g., from naturally occurring polymorphisms. Encompassed by the meaning of the term "AGXT2 gene" are thus also polynucleotides which result in a mRNA molecule exhibiting at least about 80%, 85%, 90%, 95%, 96%, 97%, 98%, 99% or more sequence identity to the sequence of SEQ ID NO: 13. Computer programs for determining the degree of identity between nucleotide sequences are available, e.g., in the Wisconsin Sequence Analysis Package, Version 8 (available from Genetics Computer Group, Madison, USA) and include, e.g., the programs BESTFIT, FASTA and GAP, which are based on the algorithm of Smith and Waterman. These programs can be used with the standard parameters, as recommended by the manufacturer. The amino acid sequence of the human AGXT2 protein is depicted in SEQ ID NO:14.

Gene expression levels can be quantified by different methods which are generally known to the skilled person. The determination of the expression of the AGXT2 gene can be performed either at the transcriptional level or, alternatively, at the translational level. Suitable methods for monitoring expression of the AGXT2 gene at the transcriptional level include those which allow for the quantitative or semi-quantitative detection of mRNA levels, for example, quantitative RT-PCR (e.g., TaqMan™ RT-PCR), real-time RT-PCR, Northern-Blot analysis or other methods which are generally described, e.g., in Sambrook et al. (eds.) Molecular Cloning: A Laboratory Manual, 2nd ed., Cold Spring Harbor Laboratory Press, 1989.

The detection of expression levels at the transcriptional level usually requires as a first step the isolation of mRNA from a biological sample of the subject, e.g. from a tissue or blood sample. Methods for isolating RNA, such as mRNA, are well known in the art and are discussed in detail in the literature (see, for example, Sambrook et al. (1989), Molecular Cloning - A Laboratory Manual, Cold Spring Harbor Laboratory, Cold Spring Harbor, New York and Ausubel et al. (1994), Current Protocols in Molecular Biology, Current Protocols Publishing, New York). Such methods regularly involve the lysis of the cells or tissues obtained from the subject to be tested. Cell lysis may be performed by use of detergents which are capable of disrupting the plasma membrane of the cells. For example, buffers containing guanidine thiocyanate and/or SDS may be used for cell lysis. The methods may comprise steps in which the DNA of the cells is enzymatically digested in order to obtain pure RNA without traces of DNA which might interfere with further downstream applications, such as the monitoring of expression levels. Inhibitors of enzymes which lead to the degradation of the RNA may also be added to the lysis buffer. Kits for preparing highly purified RNA are available from several manufacturers, such as Qiagen, Ambion, Stratagene, Clontech, Invitrogen, Promega, and others.

The RNA isolated from the cell or tissue sample by use of commercial kits will normally comprise different types of RNA. Preferably, the RNA obtained from the tissue sample is total RNA comprising mRNA, transfer RNA (tRNA) and ribosomal RNA (rRNA). For the methods of the present invention, it is desirable to enrich the mRNA fraction with respect to fractions of other cellular RNAs. Preferably, the mRNA is separated from other RNA molecules. Methods for enriching or purifying mRNA are known in the art. For example, an affinity chromatography using oligo(dT) or poly(U) coupled to a solid matrix, such as a cellulose or Sephadex™ matrix can be performed, as mRNAs contain a poly(A) tail at their 3' terminus (see, for example, Ausubel et al. (1994), Current Protocols in Molecular Biology, Current Protocols Publishing, New York). Poly(A)+ mRNA which has bound to the affinity matrix can be eluted using 2 mM EDTA/0.1% SDS.

One commonly used method for detecting expression at the transcriptional level is RT-PCR. In this method, an mRNA template is transferred into cDNA by a reverse transcription (RT) reaction. The reverse transcription of the RNA template is catalyzed by a reverse transcriptase, e.g. an avilo myeloblastosis virus reverse transcriptase (AMV-RT) or a Moloney murine leukemia virus reverse transcriptase (MMLV-RT), both of which are commonly used in commercial kits of different manufacturers. Reverse transcription can be primed by specific oligonucleotide primers, or, alternatively, by oligo-dT primers. The cDNA is then used as template for a subsequent PCR reaction. In the subsequent PCR step, the cDNA is amplified by use of specific oligonucleotide primers and a polymerase enzyme, e.g., a Taq polymerase. In a preferred aspect, the RT-PCR reaction is performed as a real-time RT-PCR, which enables the detection and the simultaneous quantification of amplified DNA in real-time. Quantification occurs either as an absolute number of copies or as a relative amount normalized by use of additional gene expression products.

In a further preferred aspect, a TaqMan RT-PCR is used for determining the expression levels of the AGXT2 gene. The TaqMan RT-PCR is a fluorophore-based RT-PCR method which detects accumulation of a specific, amplified product during PCR. In TaqMan RT-PCR, RNA is first transferred into cDNA by a reverse transcriptase. In the subsequent PCR reaction, a single-stranded oligonucleotide probe is added which is complementary to a segment of 10-60 nucleotides within the DNA template and located between the two PCR primers. A fluorophore and a quencher dye are covalently attached to the 5' and 3' ends of the probe, respectively. Alternatively, the quencher dye can also be attached to an internal nucleotide, while the fluorophore is attached to the 5' or 3' end of the probe or vice versa. The close proximity between fluorophore and quencher dye attached to the probe inhibits fluorescence emission from the fluorophore when said fluorophore is selectively excited. During DNA synthesis in the PCR, the 5' exonuclease activity of the Taq polymerase cleaves the oligonucleotide probe which is hybridized to the template DNA, thereby spatially separating the fluorophore and the quencher dye. Fluorescence is detected during PCR cycling; it is directly proportional to the fluorophore released and the amount of DNA template present in the PCR. Every fluorophore-quencher pair known in the art can be used in the methods of the present invention. Examples of suitable fluorophores are FAM (6-carboxyfluorescin), TET (tetrachlorofluo-rescin) or VIC. A suitable quencher dye is TAMRA (tetramethylrhodamine). The construction and labelling of oligonucleotides to be used as probes in a TaqMan approach are described in great detail in the literature. A TaqMan approach RT-PCR reaction can be carried out using, e.g., the ABI PRISM 7700 system (Perkin-Elmer/Applied Biosystems, Foster City, Calif., USA), or the Lightcycler system (Roche Molecular Biochemicals, Manheim, Germany).

A microarray is another commonly used tool in expression profiling. A microarray refers to the orderly arrangement of spatially resolved probes, for example nucleic acid probes, on a substrate. Such arrays can contain at least one, and preferably more than one, oligonucleotide which is complementary to the AGXT2 gene and, thus, hybridizes to the AGXT2 sequence or its transcripts. The substrate is preferably a solid substrate having a surface with a plurality of probes attached in different known locations (spots). The spots on a microarray are normally either printed on the microarrays or synthesized by photo-lithography or by ink-jet printing. On a common microarray, there may be several thousands spots, wherein each of the spots may contain a high number of identical probes, such as nucleic acid fragments or oligonucleotides. Such microarrays typically have a density of at least 100 oligonucleotides or fragments per cm². In certain embodiments the arrays can have a density of about at least 500, at least 1000, at least 10,000, at least 10⁵, at least 10⁶, at least 10⁷ oligonucleotides or fragments per cm². The support can be a glass or plastic slide or membrane on the surface of which the probes are attached at fixed locations or spots.

The primers or probes used in the PCR or RT-PCR reactions mentioned herein will be designed to allow the specific hybridization to and the subsequent amplification of the target sequence within the AGXT2 gene. Based on the present disclosure, the skilled person will be readily able to design oligonucleotide primers and/or probes which can be used for detecting the expression of the AGXT2 gene. Methods for designing sequence-specific oligonucleotide primers for PCR or RT-PCR are discussed in great detail in the scientific literature, e.g. in Dieffenbach and Dveksler, "PCR Primer", A Laboratory Manual, Cold Spring Harbor Laboratory Press, New York, 2003. The parameters to be considered when designing PCR primers include, for example, the number of nucleotides, the G/C content of the primer, the melting temperature, the presence of complementary nucleotides which may lead to secondary structures, and the like. The oligonucleotides for use in the methods of the present invention preferably have a length of at least 8 nucleotides, more preferably, at least 10, 12, 14, 16, 18, 20, 22, 24, 26, 28, 30, 32, 34, 36, 38, 40, 42, 44, 46, 48 or 50 nucleotides.

The oligonucleotide primers can be prepared by any suitable technique known in the art. For example, they may be derived synthetically, for example by the phosphoamidite method. Apart from oligomers or polymers which contain the naturally occurring nucleotide bases adenine, thymine (uridine), cytosine or guanine, oligonucleotide primers of the invention may also include modified bases, such as 5-methylcytosine, 5-hydroxymethylcytosine, xanthine, hypoxanthine, 2-aminoadenine, 6-methyladenine, 6-methylguanine, 2-thiouracil, 2-thiothymine, 2-thiocytosine, 5-halouracil, 5-halocytosine, 5-propinyluracil, 5-propinyl-cytosine, 6-azouracil, 6-azocytosine, 6-azothymine, 5-uracil, 4-thiouracil, 8-thioalkyladenine, 8-hydroxyladenine, 8-thioladenine, thioalkylguanine, 8-hydroxylguanine, 8-thiolguanine, 7-methylguanine, 7-methyladenine, 8-azaguanine und 8-azaadenine, 7-deazaguanine, 7-deazaadenine, 3-deazaguanine and/or 3-deazaadenine, and the like.

Where the determination of the mRNA levels reveals that the AGXT2 gene expression is significantly decreased in the test subject relative to the expression level in the reference subject, this indicates that the test subject has an increased mortality risk. A significant decrease of the expression level of the AGXT2 gene means that the AGXT2 mRNA amounts found in the test subject are at least about 25%, about 30%, about 35%, about 40%, about 45%, about 50%, about 55%, about 60%, about 65%, about 70%, about 75%, about 80%, about 85%, about 90%, about 95%, or at least about 100% lower than the respective amounts in the healthy reference subject. In other words, the expression level of the AGXT2 gene can be decreased in the test subject by at least 2-fold, at least 5-fold, at least 10-fold, or at least 20-fold relative to the expression level in the reference subject.

The detection of expression levels at the translational level is usually performed by measuring AGXT2 protein levels. AGXT2 protein levels may be determined by any suitable method which is able to specifically detect the AGXT2 protein in a biological sample. The detection of the protein may be based on a molecule that binds specifically to the protein or on the separation of the protein from other proteins that are present in the sample.

Molecules that specifically bind to the AGXT2 protein include antibodies and antibody fragments with binding activity for the AGXT2 protein. Such antibodies or fragments thereof may be used for detection of the AGXT2 protein in a Western blot, quantitative Western blot, enzyme-linked immunosorbent-assay (ELISA), polarization analysis, (quantitative) surface plasmon resonance (SPR), or in immunohistochemical methods, including quantitative elec-tronmicroscopic methods. Other methods which are able to detect specific binding include, for example, Förster/fluorescence resonance energy transfer (FRET). Methods which allow the quantitative detection of the AGXT2 protein by separating the protein from other components in the biological sample include quantitative mass spectrometric methods, electrophoretic methods, such as two-dimensional gel electrophoresis, and chromatographic methods, such as size-exclusion chromatography or ion-exchange chromatography.

The amounts of the AGXT2 protein measured in the sample of the test subject are indicative for the mortality risk of said subject. If the protein levels reveal that the AGXT2 gene expression is significantly decreased in the test subject compared to the reference subject, this indicates that the test subject has an increased mortality risk. A significant decrease of the expression level of the AGXT2 gene means that the AGXT2 protein amounts found in the test subject are at least about 25%, about 30%, about 35%, about 40%, about 45%, about 50%, about 55%, about 60%, about 65%, about 70%, about 75%, about 80%, about 85%, about 90%, about 95%, or at least about 100% lower than the respective amounts in the healthy reference subject. The protein amounts in the test subject can be decreased by at least 2-fold, at least 5-fold, at least 10-fold, or at least 20-fold relative to the corresponding amounts in the reference subj ect.

### Diagnostic methods targeting enzymatic activity

In a further aspect, the invention relates to a method which compares the activity of the AGXT2 enzyme as measured in a sample from a test subject to a reference value that was obtained from a healthy person, i.e. a person who does not show any clinical signs for a cardiovascular disease, such as a stroke.

The method comprises
(a) detecting in a sample from said test subject the activity of the alanine-glyoxylate-aminotransferase 2 (AGXT2) enzyme;
(b) comparing said activity of step (a) to the activity of said enzyme in a reference subject;
wherein detection of an activity of the AGXT2 enzyme which is significantly decreased in the test subject relative to the activity in the reference subject indicates that the test subject has an increased mortality risk. Preferably, the sample in which the activity of the AGXT2 enzyme is detected is a kidney, liver or blood sample, more preferably a blood plasma sample.

Enzymatic AGXT2 activity may be determined in a suitable assay, e.g. by measuring the reduction rate of educts of the AGXT2 reaction, preferably L-alanine and/or SDMA, or by measuring the product formation rate, preferably of pyruvate, pyruvate derivatives and di-methylguanidino valeric acid. The educts of the reaction may be labelled for the purpose of a simplified detection of the product of the reaction. The educts may for example be labelled with stable isotopes or radioisotopes, such as ²H, ¹³C, ¹⁵N, ¹⁸O, ³H and ¹⁴C, and monitored during reaction by mass spectrometric detection or scintillation detection. This method has been previously described in detail for stable isotopes by Maas et al. ((2007), Chromatogr B Analyt Technol Biomed Life Sci., 851:220-8). Such an enzyme activity assay is based on scintillation detection for radioisotopes and has been previously described in detail by MacAl-lister et al. ((1996), Br J Pharmacol., 119:1533-40). In a preferred embodiment, the assay for determining the activity of the AGXT2 enzyme measures the degradation of d6-SDMA in the presence of AGXT2. The enzyme activity assays for detecting enzymatic AGXT2 activity are preferably carried out with the human AGXT2 enzyme depicted in SEQ ID NO:14 (NM_031900.1; Protein ID: EC 2.6.1.44). Alternatively, an enzymatically active fragment or homolog of the human enzyme shown in SEQ ID NO:14 may be used. As used herein, a homolog of the sequence of SEQ ID NO:14 refers to a polypeptide molecule having a high degree of sequence identity with the amino acid sequence of SEQ ID NO:14. amino acid identity will be at least about 75%, 80%, 85%, 90%, 91%, 92%, 93%, 94%, 95%, 96%, 97%, 98% or 99% when compared in an optimal alignment, for example, by the program BESTFIT using standard parameters.

The assays used according to the present invention may also be based on one or more enzymatically active fragments of the human AGXT2 enzyme shown in SEQ ID NO:14 or on enzymatically active fragments of the above-mentioned homologs. Enzymatically active fragments of the sequence shown in SEQ ID NO:14 or its homologs are polypeptides that differ from the amino acid sequence shown in SEQ ID NO:14 (or from the respective homolog sequence) by the absence of one or more amino acids at the N-terminus and/or the C-terminus of the polypeptide. For example, a fragment of the sequence of SEQ ID NO:14 may differ from the sequence of SEQ ID NO:14 by the lack of about 5, 10, 15, 20, 25, 30, 35, 40, 45 or 50 amino acids at the N-terminus and/or the C-terminus, provided that such fragment retains at least a part of the enzymatic activity of the original full-length enzyme depicted in SEQ ID NO:14. Likewise, a fragment of a homolog of SEQ ID NO:14 may differ from said homolog sequence by the lack of about 5, 10, 15, 20, 25, 30, 35, 40, 45 or 50 amino acids at the N-terminus and/or the C-terminus, provided that the fragment is still enzymatically active.

A significant decrease in the AGXT2 enzyme activity means that the AGXT2 activity levels found in the test subject are at least about 25%, about 30%, about 35%, about 40%, about 45%, about 50%, about 55%, about 60%, about 65%, about 70%, about 75%, about 80%, about 85%, about 90%, about 95%, or at least about 100% lower than the respective levels in the reference subject. The activity levels in the test subject can be decreased by at least 2-fold, at least 5-fold, at least 10-fold, or at least 20-fold relative to the corresponding levels in the reference subject.

### Diagnostic methods targeting SDMA levels

In still a further aspect, the invention relates to a method for determining/predicting the all-cause mortality risk of a test subject, comprising
(a) detecting in a sample from said test subject the concentration of SDMA;
(b) comparing the concentration of step (a) to the concentration of SDMA in a sample of a reference subject;
wherein detection of a SDMA concentration which is significantly increased in the test subject relative to the concentration in the reference subject indicates that the test subject has an increased mortality risk. The sample in which the concentration of the SDMA is detected preferably is a liver, kidney or blood sample, more preferably a blood plasma sample.

The concentration of SDMA in the sample, e.g. in a blood plasma sample, can be detected by several methods known in the art. These methods include, for example, immunoassay-based methods, such as enzyme-linked immunosorbent-assay (ELISA) and radioimmunoassay (RIA). Chromatographic methods for the detection of SDMA in the blood plasma include for example high performance-liquid chromatography (HPLC), tandem liquid chromatography-mass spectrometry (LC-MS/MS), tandem gas chromatography-mass spectrometry (GC-MS/MS), and the like. In a particularly preferred embodiment, the concentration of SDMA is detected by LC-MS/MS in human plasma as described elsewhere (Schwedhelm et al. (2005), Clin Chem, 51:1268-71, and Schwedhelm et al. (2007), J Chromatogr B Anal Technol Biomed Life Sci, 851:211-9). In a further particularly preferred embodiment, the concentration of SDMA is detected by high performance liquid chromatography and fluorescence detection after derivatization with o-phthalaldehyde as described previously (Teerlink T, et al. (2002), Anal Biochem. 303:131-7). In a another preferred embodiment, the concentration of SDMA is detected by GC-MS/MS using stable isotope-labeled SDMA as internal standard (Tsikas et al. (2011) Anal Biochem., 413:60-2).

The reference subject will preferably exhibit a SDMA concentration in the plasma which is typical of the general population of normal, disease-free subjects. In healthy humans, the mean plasma concentration of SDMA is in the range of 0.225 to 0.533 µmol/L (2.5^{th} and 97.5^{th} percentile of a reference population). The SDMA concentration is significantly increased in the test subject, if the SDMA concentration in the test subject is higher than 0.533 µmol/L, preferably higher than 0.7 µmol/L, 0.9 µmol/L, or 1.1 µmol/L.

### Diagnostic methods targeting genomic DNA

In another aspect, the present invention relates to methods for assessing the all-cause mortality risk of the subject by detecting alterations in the genome of a subject that result in a decreased expression of the AGXT2 gene and/or a reduced activity of the AGXT2 enzyme.

Specifically, the present invention provides a method for determining/predicting the all-cause mortality risk of a test subject, comprising detecting in a biological sample a genetic alteration in the genome of said test subject, wherein said genetic alteration is associated with a decreased expression of the AGXT2 gene and/or with a decreased activity of the AGXT2 enzyme. The detection of such a genetic alteration indicates that the test subject has an increased mortality risk.

The genetic alteration referred to herein may be any kind of alteration in the genome of the test subject that is capable of interfering with the expression of the AGXT2 gene, such as inactivating insertion, deletions of one or more nucleotides in the coding region, mutations of the promoter structure, single nucleotide polymorphisms, loss of gene fragments and the like. The genetic alteration can be located within intronic sequences or the coding sequence of the AGXT2 gene or in a region flanking said coding sequence. The alteration may either completely or partially decrease the expression of the AGXT2 gene. Alternatively, the alteration may completely or partially inactivate the AGXT2 enzyme, i.e. lead to a decreased activity of the enzyme compared to the wild type enzyme.

In a preferred embodiment, the detection of the genetic alteration which is associated with a decreased expression of the AGXT2 gene and/or with a decreased activity of the AGXT2 enzyme comprises the detection of a single nucleotide polymorphism (SNP). As used herein, a SNP refers to a nucleotide position in a nucleic acid, such as a DNA or RNA, which is occupied by alternative nucleotides in different alleles in a population. The position of the SNP is usually flanked by highly conserved sequences of the allele. An individual, such as a human, normally has two alleles and therefore may be homozygous or heterozygous with respect to a given allele at a particular SNP position. In a biallelic organism, for example in a human, a single nucleotide polymorphism can result in three different genotypes. For example, an A/C SNP marker can result in a homozygous genotype "AA", a homozygous genotype "CC" or a heterozygous genotype "AC". Most SNPs have only two alleles, i.e. one group of the population exhibits a specific nucleotide at the SNP position, whereas the other group exhibits a different nucleotide at the same position.

A SNP can be located in a coding region of the AGXT2 gene, in a non-coding region or in an intergenic region. Where the SNP is located in the coding region of the AGXT2 gene, it may be synonymous or non-synonymous. A synonymous or silent nucleotide substitution is one that due to the degeneracy of the genetic code does not result in an amino acid change. In contrast, a non-synonymous nucleotide substitution yields in an amino acid substitution and frequently gives rise to an altered expression product. Alternatively, it may lead to a premature termination of the translation process if a stop codon is formed by the substitution at the polymorphic site. It is also possible that the substitution destroys a naturally occurring stop codon at the polymorphic site which leads to an extended read-through expression product. In contrast, where a SNP is not in a coding region of the genome, it may nevertheless affect protein expression, for example by influencing RNA splicing or DNA binding to transcription factors, and the like.

The present invention provides a number of SNPs in the AGXT2 gene and in the vicinity of the AGXT2 gene which have been found to be associated with a high SDMA concentration in the serum of individuals, and therefore with an increased mortality risk. The SNPs are listed in the table below. The nucleotides in brackets indicate the polymorphism. The first nucleotide in brackets represents the major, the second the minor allele. The major allele is the non-risk allele, the minor allele is the risk allele, i.e. the nucleotide which has been identified in persons having a significantly higher SDMA plasma concentration.

**Table 1: SNPs associated with increased SDMA plasma concentration. Position 27: Major/Minor Allele.**

| | |
|---|---|
| rs28305 | CCTCCATGGGAGGCAGTGTCTCAAAC[G/C]TCAATATCAGTGAGGGACTTCAAAA (SEQ ID NO:1) |
| rs37369 | TTCATGCATTGGAGGGTGGAAGAAGA[C/T]GGTGCTTGTATGCCACAGGCGGCCG (SEQ ID NO:2) |
| rs1428281 | GTTTTAAAAAATAAGTCATTTAATAA[T/C]GTTTGGCAATTTCCTTTTAATAATA (SEQ ID NO:3) |
| rs37370 | TCCCGGAAAAGAAATCCAGGTATCTG[T/C]TTCCTTCAGCATCAAAGAGCCACTC (SEQ ID NO:4) |
| rs40200 | GTGTGTGTTTGGATGAGCAAGCACAA[G/A]CAGGCACAGACCTAGGTAGAGTAGG (SEQ ID NO:5) |
| rs37379 | TTTTTGCAAGGCCAGGTGATGAGGAT[G/A]GCATTCAGTCTAGGTAGTGTGGTTT (SEQ ID NO:6) |
| rs163594 | AGGAGGTAGGAGGAGAGTGGAAATTA[T/G]GCCAGAAAGAAAATATGTTTTTTAA (SEQ ID NO:7) |
| rs10941225 | TGAAGATCAGTGATCCTTAATTACCT[A/T]AGAAGGCTCAGTACATTTTTACTTT (SEQ ID NO:8) |
| rs153009 | CTAGGTCGCTGAACACATCCACATGT[T/C]GGGAGAGTAGCACACCCCAGCATGG (SEQ ID NO:9) |
| rs163585 | CTCTGGACGCTTCCAGACCTTGCCCT[A/G]TACACCTCTTCTTCTGGCTGTTCAT (SEQ ID NO:10) |
| rs163588 | ATCTACTTTTATTCCTGTTCCCACTT[T/C]TGTTCAGGTATCCATCCTAAGCGGA (SEQ ID NO:11) |

In a preferred embodiment, the diagnostic method of the invention which makes use of polymorphisms in the genomic DNA of individuals suffering from an increased mortality risk comprises the step of detecting the presence or absence of at least one nucleotide exchange from a non-risk to a risk allele depicted in Table 1 in a nucleic acid sample of the test subject, wherein a change from the non-risk allelic form to the risk allelic form is indicative for an increased mortality risk. The detection of a homozygous genotype of the risk allele in a subject generally indicates a higher risk than the detection of the corresponding heterozygous genotype.

The detection of a specific nucleotide at a SNP site in a polynucleotide is commonly referred to as genotyping of the SNP. Numerous methods are described in the prior art which provide for genotyping of SNPs in genomic sequences. See for example, Chen et al, Pharmacogenomics J. (2003), 3(2):77-96; Kwok et al., Curr. Issues Mol. Biol. (2003), 5(2):43-60; Kwok, Annu. Rev. Genomics Hum. Genet. (2001), 2:235-58; Shi, Am. J. Pharmacogenomics (2002), 2(3):197-205. For example, methods for SNP genotyping that can be used according to the present invention include TaqMan assays, molecular beacon assays, nucleic acid microarrays, primer extension techniques and oligonucleotide ligation assays. These methods may be used in combination with different labeling techniques, including fluorescence, luminescence or chemiluminescence labeling, and the like. Preferably, the genotyping methods are suitable for a high throughput format and accessible to automation.

In a simple embodiment, genomic DNA is amplified by PCR methods. For this, genomic DNA is firstly isolated from a sample of the test subject. A variety of different sample types may be used, provided that the sample contains cells containing genomic DNA of the test subject. The sample may be a tissue sample obtained from the test subject, e.g., by core needle biopsy, fine needle aspiration, and the like. In a preferred embodiment, the biological sample is a blood sample, and the genomic DNA is extracted from white blood cells, such as lymphocytes, monocytes and/or neutrophils. Several methods for isolating genomic DNA from eukaryotic cells or tissues have been described in the art. The genomic DNA may for example be isolated as described by Sambrook et al., Molecular Cloning: A Laboratory Manual, CSH Press, 2nd edition (1989). More conveniently, commercially available kits can be used for isolating genomic DNA, for example, the QIAamp DNA mini kit (Qiagen) or the ChargeSwitch® Genomic DNA Purification Kit (Invitrogen). Methods for isolating DNA usually involve the lysis of the cells of the cellular or tissue sample obtained from the test subject. Cell lysis may be performed by disrupting the plasma membrane of the cells, for example, by boiling the sample or by use of detergents such as SDS or similar compounds. The RNA of the sample is normally digested by using RNAse. Protein contaminants can be readily degraded by incubating the sample with proteinase K. Genomic DNA can be recovered by alcohol precipitation or by binding to an anion exchange column.

PCR-based methods are then used to amplify a nucleic acid sequence comprising the putative SNP site of interest. Briefly, PCR primers which specifically hybridize to upstream and downstream regions of the SNP site in the nucleic acid to be investigated can be used for the amplification. The PCR products obtained in this manner can then be subjected to common sequencing techniques, e.g. by a method based on the chain termination technique described by Sanger (J. Molecular. Biol. 94:441 (1975)). The PCR used for SNP genotyping may be a multiplex PCR. Such a multiplex PCR is performed by using a plurality of primers and genomic DNA as template for amplifying the nucleic acid sequences containing the SNP(s) to be detected.

In a preferred embodiment of the present invention, a TaqMan^{™} PCR approach is used for SNP genotyping. The basic principles of the TaqMan^{™} PCR approach are discussed elsewhere herein. It is preferred to use differently labeled probes in the same TaqMan^{™} assay. For example, one TaqMan^{™} probe specific for the risk allele of the SNP is labeled with FAM and another TaqMan^{™} probe that is specific for the non-risk allele is labeled with VIC. In such a set-up, fluorescence of FAM and no fluorescence of VIC would indicate the presence of the risk allele, whereas fluorescence of VIC and no fluorescence of FAM would be indicative for the presence of the non-risk allele. Fluorescence of both, FAM and VIC, would be indicative for a heterozygous state. The present invention also contemplates modifications of the TaqMan^{™} method, for example, assays that use molecular beacons.

A further method for detecting a SNP in a polynucleotide sequence is described, for example, in US 4,988,617. In this method, the occurrence of a specific nucleotide is determined by an oligonucleotide ligation assay using a pair of oligonucleotide probes that selectively hybridize immediately upstream and downstream of the polymorphic site. Upon hybridization, one of the terminal nucleotides of the oligonucleotides is aligned to the site of the putative polymorphism. Where the terminal nucleotide of the probe is complementary to the nucleotide at the polymorphic site, hybridization of the oligonucleotide to the polynucleotide that contains the polymorphic site will include the terminal nucleotide of said oligonucleotide. In this case, the addition of a ligase, such as a T4 ligase, will link both oligonucleotide probes to each other. In contrast, where the terminal nucleotide of the probe is not complementary to the nucleotide at the polymorphic site, no ligation of the oligonucleotide to the polynucleotide containing the polymorphic site will occur. The ligation product derived from the linkage of the two oligonucleotides can be detected in a subsequent step, for example by a PCR reaction. Several test systems are currently available which are based on oligonucleotide ligation.

Single base extension methods can also be used for the detection of SNPs. Single base extension methods are described, e.g., in US 5,846,710, US 6,004,744, US 5,888,819 and US 5,856,092. Briefly, these methods are based on the hybridization of a primer that is complementary to a target sequence such that the 3' end of the primer is adjacent to but does not span the site of the putative SNP in the target sequence. The primer comprises a sequence which is complementary to the target polynucleotide terminating at the base that is immediately adjacent 5' to the polymorphic site. The hybridization is performed in the presence of one or more labeled nucleotides complementary to base(s) that may occupy the site of potential variation. For example, for a biallelic polymorphism two differentially labeled nucleotides can be used. For a tetra-allelic polymorphism four differentially labeled nucleotides can be used. Preferably, the labeled nucleotides are dideoxynucleotides. Hybridization is performed under conditions permitting primer extension if a nucleotide complementary to a base occupying the site of variation in the target sequence is present. Extension incorporates a labeled nucleotide thereby generating a labeled extended primer. If multiple differentially labeled nucleotides are used with a heterozygous target, multiple differentially labeled extended primers are obtained. Extended primers are detected providing an indication of which base(s) occupy the polymorphic site in the target sequence.

Another embodiment of the invention refers to genotyping by an invader assay (Olivier M., Mutat Res. (2005), 573(1-2), 103-10). In the invader assay, a cleavase (e.g., the flap endonuclease FEN) is combined with two specific oligonucleotide probes that together with the target DNA can form a tripartite structure recognized by the cleavase. The first probe, called the invader oligonucleotide is complementary to the 3' end of the target DNA. The last base of the invader oligonucleotide is a non-matching base that overlaps the SNP nucleotide in the target DNA. The second probe is an allele-specific probe which is complementary to the 5' end of the target DNA, but also extends past the 3' side of the SNP nucleotide. The allele-specific probe will contain a base complementary to the SNP nucleotide. If the target DNA contains the desired allele, the invader and allele-specific probes will bind to the target DNA forming the tripartite structure. This structure is recognized by cleavase, which will cleave and release the 3' end of the allele-specific probe. If the SNP nucleotide in the target DNA is not complementary to the allele-specific probe (risk or non-risk allele), the correct tripartite structure is not formed and no cleavage occurs. The invader assay can be coupled with a fluorescence resonance energy transfer (FRET) system to detect the cleavage. In this case, a quencher molecule is attached to the 3' end and a fluorophore is attached to the 5' end of the allele-specific probe. If cleavage occurs, the fluorophore will be separated from the quencher molecule generating a detectable signal.

According to another embodiment, the genotyping of the SNPs is carried out by a hybridization assay using a suitable probe or suitable probes. Any standard hybridization assay known to the person skilled in the art can be used in the methods of the present invention. In a preferred embodiment a dynamic allele-specific hybridization approach is used. This approach is based on the differences in the melting temperature in DNA that results from the instability of mismatched base pairs. In the first step of dynamic allele-specific hybridization, a genomic fragment comprising the SNP is amplified and attached to a bead through a PCR reaction with a biotinylated primer. In the second step, the amplified product is attached to a streptavidin column and washed (e.g. with NaOH) to remove the nucleic acids which are not biotinylated. An allele specific oligonucleotide is then added in the presence of a molecule that emits fluorescence when bound to double-stranded DNA. The intensity is then measured as temperature is increased until the Tm (melting temperature) can be determined. If the probe is specific for the non-risk allele of the SNP, occurrence of the risk allele will result in a lower than expected Tm and therefore, the risk allele can be detected (Howell W., et al., Nat. Biotechnol. (2005), 17(1), 87-8).

### Screening for compounds for decreasing the mortality risk

The insight that a decreased expression of the AGXT2 gene and/or a decreased activity of the AGXT2 enzyme increase the mortality risk of a subject allows the design of screening assays which identify pharmaceutically active compounds that are effective in decreasing the mortality risk of a subject. The present invention thus also provides novel methods for screening for compositions which modulate the mortality risk of a subject. The screening methods comprise the step of monitoring the expression level of the AGXT2 gene or, alternatively, the activity of the AGXT2 enzyme in the presence of candidate compounds. Any increase in the expression level of the AGXT2 gene and/or the activity of the AGXT2 enzyme in the presence of a candidate compound relative to the expression level and/or the enzymatic activity in the absence of said candidate compound is an indication that the compound is effective in decreasing the mortality risk by decreasing the SDMA concentration in the plasma of an individual.

Thus, the invention provides an *in vitro* method for identifying a pharmaceutically active compound which decreases the mortality risk of a subject, preferably a human subject, comprising
(a) contacting a candidate compound with a cell, preferably a human cell, that expresses the alanine-glyoxylate-aminotransferase 2 (AGXT2) gene;
(b) detecting whether said candidate compound alters the expression level of the AGXT2 gene;
wherein an increase of the expression level of the AGXT2 gene in the presence of the compound (relative to the expression level in the absence of said compound) indicates a pharmaceutically active compound.

In another aspect, the invention provides an *in vitro* method for identifying a pharmaceutically active compound which decreases the mortality risk of a subject, preferably a human subject, comprising
(a) contacting a candidate compound with an alanine-glyoxylate-aminotransferase 2 (AGXT2) enzyme;
(b) detecting whether said candidate compound alters the activity of the AGXT2 enzyme;
wherein an increase of activity of the AGXT2 enzyme in the presence of the compound (relative to the activity in the absence of said compound) indicates a pharmaceutically active compound.

A number of different assay designs to evaluate the effects of candidate compounds on gene expression or enzyme activity may be used. The screening methods are preferably carried out as high throughput screening techniques which allow for the examination of thousands of different compounds in a short period of time. High throughput screening techniques are described in detail in the prior art. The compounds identified by the screening methods can be validated in animal models, such as mouse models to confirm their activity *in vivo.*

The candidate compounds used in the screening methods of the present invention can include all different types of organic or inorganic molecules, including peptides, oligo- or polysaccharides, fatty acids, steroids, and the like. Typically, the candidate compounds will be small molecules with less than about 2,500 daltons, less than 2000 daltons, less than 1500 daltons, less than 1000 daltons, or less than 500 daltons. Candidate compounds for use in screening methods can be provided in the form of libraries which comprise a high number of synthetic or natural compounds.

In a preferred embodiment, a candidate compound is considered potentially active if it increases the AGXT2 expression level or enzyme activity by at least 1%, 5%, 10%, 20%, 30%, 40%, 50%, 60%, 70%, 80%, 90%, 100%, 150%, 200%, 250%, or greater compared to the expression or activity in the absence of the compound. In some embodiments the increase in the AGXT2 expression level or enzyme activity will be 300%, 400%, 500%, 600%, 700%, 800%, 900% or even 1000% or greater compared to the expression or activity in the absence of the compound.

### Therapeutic approaches for decreasing the mortality risk

Apart from the predictive methods described above, the present invention also provides a method for decreasing the mortality risk of a subject, comprising administering to said subject a composition or compound which is effective in increasing the expression of the AGXT2 gene and/or the activity of the AGXT2 enzyme in said subject. The invention therefore also relates to a composition or compound which is effective in increasing the expression of the AGXT2 gene and/or the activity of the AGXT2 enzyme for use in a method of decreasing the mortality risk of a subject.

One way of complementing a pathologically decreased expression of the AGXT2 gene and/or a pathologically decreased activity of the AGXT2 activity in a subject is to provide said subject with a functional copy of the AGXT2 gene, e.g. by gene therapy. A gene therapy approach facilitates the insertion of a functional gene into an unspecific or specific location of the host genome. For this purpose, the AGXT2 gene is cloned into a viral or non-viral vector. The AGXT2 gene may be operably linked to a promoter element and, optionally, to an enhancer element. A suitable promoter is, e.g., the immediate early promoter of the cytomegalovirus (CMV). The promoters and enhancers may further be selected from those known in the art, such as the promoters and enhancers of the LTR of Rous sarcoma virus, the TK gene of HSV-1, the early or late promoters of SV40, the adenovirus major late promoter (MLP), phosphoglycerate kinase genes, metallothionein genes, a-1 antitrypsin genes, albumin genes, collagenase genes, elastase I genes, β-actin genes, β-globin genes, γ-globin genes, α-fetoprotein genes, and muscle creatin kinase genes.

Where the subject to be treated by gene therapy is a human, the vector to be administered typically is a viral vector. Typically, viral vectors are able to infect the target cells and introduce the gene of interest into the cell. The viral vectors may either be endogenously able to infect the target cell or be engineered to do so. Such engineering may be that the viral vector contains a ligand molecule in its viral capsid and/or envelope which binds to surface molecules of the target cells.

Suitable viral vectors for use in the present invention are recombinant DNA or RNA viruses, more preferably replication-deficient viruses, and include, e.g., detoxified retrovirus, adenovirus, adeno-associated virus (AAV), herpes virus, poxvirus, vaccinia virus, poliovirus, Sindbis virus, polyomavirus, such as simian virus 40 (SV40), human immunodeficiency virus (HIV), and others.

Adenoviruses are particularly preferred, as the transduction efficiency is typically higher with adenoviruses compared to other viruses. The adenovirus may be a human adenovirus type 5 (hAd5) vector, an E1-deleted and/or an E3-deleted adenovirus. For example, an adenoviral vector can be constructed by the rescue recombination technique as described in McGrory, et al. (1988), Virology 163:614-617. Briefly, the transgene of interest is cloned into a shuttle vector that contains a promoter, a polylinker and flanking adenovirus sequences from which E1A/E1B genes have been deleted. Suitable shuttle vectors include, e.g., the plasmid "pAC1 " (McGrory, et al. (1988), Virology 163:614-617) which encodes portions of the left end of the human adenovirus 5 genome but which lacks the early protein region comprising E1A and E1B sequences that are essential for viral replication. Another suitable shuttle plasmid is "ACCMVPLPA" (Gomez-Foix et al. (1992), J. Biol. Chem. 267: 25129-25134) which contains a polylinker, CMV promoter and SV40 polyadenylation signal flanked by partial adenovirus sequences from which the E1A/E1B genes have been deleted. The shuttle plasmid can be co-transfected, e.g., by lipofection or calcium-phosphate-transfection, along with a plasmid comprising the entire human adenovirus 5 genome with a length that is too large to be encapsidated into suitable host cells (e.g., human 293 cells). In a subset of cells "rescue recombination" between the shuttle vector and the helper plasmid will occur, creating a plasmid which contains the gene of interest in the place of the E1A/E1B genes and of the additional sequences which previously rendered the plasmid too large to be encapsidated. This can be monitored, e.g., with the beta-galactosidase/x-gal system which is well known in the art. The resulting plasmid of interest will be small enough to be encapsidated but replication deficient (see, e.g., Giordano et al. (1996), Nature Medicine 2: 534-539).

In another embodiment, the viral vector may be a poxvirus, e.g. a vaccinia virus, preferably an attenuated vaccinia virus, an avipox virus or an attenuated avipox virus, or a swinepox, rac-coonpox, camelpox, or myxomatosis virus. In a vaccinia virus, the insertion site or sites for the gene of interest may be advantageously at the thymidine kinase (TK) gene or insertion site, the hemagglutinin (HA) gene or insertion site, or the region encoding the inclusion body of the A type (ATI). In yet another embodiment the viral vector may be a herpes virus such as a canine herpes virus (CHV) or a feline herpes virus (FHV). Suitable insertion sites for the gene of interest are known in the art.

Recombinant viral vectors can be plaque-purified according to standard techniques. For example, recombinant adenoviral vectors can be propagated in human 293 cells (which provide E1A and E1B functions *in trans*) to titers in the range of 10⁷-10¹³ viral particles/mL). Prior to *in vivo* application viral vectors may be desalted by gel filtration methods, such as Sepharose columns, and purification by subsequent filtering. Purification reduces potential deleterious effects in the subject to which the vectors are administered. The administered virus is substantially free of wild-type and replication-competent virus. The purity of the virus can be proven by suitable methods, such as PCR.

Non-viral expression vectors may also be used for introducing a functional AGXT2 gene into a human subject. Suitable expression vectors permit the *in vivo* expression of the AGXT2 gene in the target cell. Examples for non-viral expression vectors include vectors such as pCAGGS (Niwa et al. (1991), Gene, 108: 193-200), pBK-CMV, pcDNA3.1, pZeoSV (Invitrogen, Stratagene). These vectors may be administered, for example, by direct injection or non-invasive catheter or injector methods. Alternatively, target cells that have been removed from a subject, for example, by a biopsy procedure, may be transfected with the vector construct in an *ex vivo* procedure. The cells can then be implanted or otherwise administered into a subject, preferably into the subject from whom they were obtained. Suitable methods for the transfer of non-viral vectors into target cells are for example the lipofection method, calcium-phosphate co-precipitation method, DEAE-dextran method and direct DNA introduction methods using micro-glass tubes and the like.

Prior to the introduction of the vector, the target cells and/or tissues may be treated with a permeabilization agent selected from the group consisting of SDS and SLS and their derivatives, phosphatidylcholine, streptolysins, equinatoxins, methyl-beta-cyclodextrin, sodium caprate, decanoylcamitine, tartaric acid, lysolecithin, polyoxyethylenesorbitan monolaurate, octylphenoxy polyethoxy ethanol, t-octylphenoxypolyethoxyethanol (Triton X-100), alame-thicin, Saponin, non-ionic detergents (NPs, such as NP-40) or Tween and its derivatives. Target cells may also be permeabilized by ultrasonic shock, osmotic shock or electric pulses.

Another therapeutic approach for decreasing the mortality risk of a subject is to administer to a subject in need thereof a compound, such as a small molecule, which is effective in increasing the expression of the AGXT2 gene and/or the activity of the AGXT2 enzyme in order to decrease the mortality risk. Preferably, the compound has been obtained by screening of a library, such as a small molecule library, as described elsewhere herein. The compounds which are identified by applying the screening methods disclosed herein may be further modified by molecular modelling approaches and subsequent chemical synthesis of the desired molecule. In this way, the binding affinity for AGXT2, the potency to increase the AGXT2 expression and/or activity or other physiological properties of the compound may be improved. Suitable methods to assist in a strategic modification of a compound include the determination of the three-dimensional structure of the compound in complex with its binding partner by Nuclear-Magnetic Resonance (NMR) or X-ray crystallography and subsequent computer-based modelling of suitable compound-modifications. Strategically modified compounds will be synthesized and also tested in above described methods.

Suitable compounds to increase the activity of the AGXT2 enzyme are known co-factors of AGXT2 (e.g. known forms of vitamin B6). The term "vitamin B6" comprises pyridoxalphosphate, pyridoxinphosphate, and a group of compounds such as 4,5-Bis(hydroxymethyl)-2-methyl-3-pyridinol (Pyridoxin), 3-Hydroxy-5-hydroxy-methyl-2-methyl-4-pyridin-carbaldehyde (Pyridoxal), and 4-(Aminomethyl)-5-hydroxy-6-methyl-3-pyridinmethanol (Pyridoxamin). These compounds have an excellent safety profile and can thus be considered suitable for clinical applications.Another compound that is specifically contemplated by the present invention for increasing the expression of the AGXT2 gene is (R)-b-aminoisobutyric acid (CAS Registry Number 2140-95-6, 3-amino-2-methylpropanoic acid, 3-amino-2-methylpropanoic acid, D-2-methyl-b-Alanine) and structurally or functionally related derivatives thereof. (R)-b-aminoisobutyric acid can thus be used to decrease SDMA concentrations in a patient to be treated.

The compound, e.g. the small molecule, which is effective in increasing the expression of the AGXT2 gene and/or the activity of the AGXT2 enzyme can then be formulated into a pharmaceutical composition for administration to the subject in need thereof. Methods for the preparation of such pharmaceutical compositions are well known by those working in the field of pharmaceutics. Typically, such compositions are prepared either as liquid solutions, powders or suspensions. The pharmaceutical composition of the invention can include commonly used pharmaceutically acceptable excipients, such as diluents and carriers. In particular, the composition comprises a pharmaceutically acceptable carrier, e.g., water, saline, Ringer's Solutions, or dextrose solution. Further examples of suitable carriers are described in standard textbooks, for example, in "Remington's Pharmaceutical Sciences", Mack Pub. Co., New Jersey (1991). In addition to the carrier, the composition may also contain wetting agents, emulsifying agents, pH buffering agents, stabilizers, dyes and the like.

The pharmaceutical composition provided by the present invention will be formulated to be compatible with the intended route of administration. Different routes of administration are feasible for providing the pharmaceutical composition to a subject in need thereof. The pharmaceutical composition may be formulated for parenteral administration, for example, for intravenous, intrathecal, intradermal, subcutaneous, or rectal administration. The composition can be enclosed, e.g. in ampoules, disposable syringes or multiple dose vials made of glass or plastic.

The pharmaceutical composition of the invention may be formulated for oral administration, for example, in the form of granules, powders, tablets, pills, or capsules. The preparation of solid dosage forms can be achieved by mixing the active ingredient with an appropriate edible carrier, such as microcrystalline cellulose, methylcellulose, hydroxypropyl methylcellulose, casein, albumin, mannit, dextran, saccharose, lactose, sorbitol, agar, alginate, pectine, collagen, starch or gelatine. The solid dosage form may also include surfactants, binders, thickeners, diluents, lubricants, disintegrants, and glidants. Further, the solid dosage form can comprise antioxidants (for example, ascorbic acid, tocopherol or cysteine), preservatives (for example, parabene), coloring or flavoring agents, and the like. Mixing of the above components may involve dry granulation, wet granulation, or direct compression.

The pharmaceutical composition of the invention will comprise a therapeutically effective amount of the active ingredient. A therapeutically effective amount of the active ingredient is typically an amount such that, when administered in a physiologically tolerable composition, is sufficient to reduce an elevated plasma concentration of SDMA in the patient from above 0.7 µmol/l to below 0.55 µmol/l, preferably from above 1.1 µmol/l to below 0.55 µmol/l, wherein a target level within the lower range of the normal distribution i.e. 0.1-0.5 µmol/l is particularly preferred. Thus, a therapeutically effective amount of the active ingredient is typically an amount such that, when administered in a physiologically tolerable composition, is sufficient to achieve a plasma concentration of SDMA in the patient from about 0.7 µmol/l to about 0.1 µmol/l, preferably from about 0.5 µmol/l to about 0.1 µmol/l. Therefore, according to a preferred embodiment, the dosage of the active ingredient per body weight can vary from about 0.01 mg per kg body weight of the subject to about 400 mg per kg body weight of the subject, preferably from about 0.15 mg per kg body weight of the subject to about 200 mg per kg body weight of the subject, and more preferably from about 0.5 mg per kg body weight of the subject to about 200 mg per kg body weight of the subject, used in one or more dose administrations daily. Thus, according to a preferred embodiment of the invention, the therapeutically effective amount of the active ingredient ranges from 0.15 mg per kg body weight of the subject to about 400 mg per kg body weight of the subject.

The active ingredient can be orally supplemented in a manner similar to that previously described for arginine, or lysine. Oral supplementation of active ingredients has been performed exemplary in a clinical investigation in humans with arginine and citrulline (Schwedhelm E, et al. (2008) Br J Clin Pharmacol., 65:51-9.).

### EXAMPLES

The aim of the experiments described herein was to evaluate the association of SDMA levels with the outcome in patients after acute ischemic stroke and, at the same time, elucidate the enzymatic pathways involved in the regulation of SDMA plasma concentration.

A well characterized secondary outcome cohort of ischemic stroke patients was studied in view of a potential association with SDMA (see Example 1 below). By applying genome-wide association (GWA) analysis, an association of SDMA plasma concentration with certain types of genetic alterations was established (see Example 2 below). In an experimental model of the enzymatic pathway of AGXT2 it was shown that kidney tissue lysate metabolizes SDMA to form pyruvate (see Example 3 below). Additionally, the association between the AGXT2 genotype and stroke outcome was investigated (see Example 4 below). Finally, it was demonstrated in HEK cells transfected with a wild-type AGXT2 gene or an AGXT2 variant, that SDMA levels are causally related to the presence of a functional AGXT2 enzyme (see Example 5 below).

### EXAMPLE 1: Association of SDMA with stroke outcome

For examining a potential association between the serum concentration of SDMA and outcome in stroke patients, the Leeds Stroke Study was used. The recruitment and characteristics of patients in the Leeds Stroke Study have been fully described elsewhere (Carter et al. (2007), Stroke, 38(6): 1873-80). Briefly, European patients (n=609) with a clinical diagnosis of acute ischemic stroke were recruited from four hospitals in Leeds. Ischemic stroke was classified according to the Oxfordshire Community Stroke Project classification, as lacunar infarction (LACI), total and partial anterior circulation infarction (TACI, PACI), and posterior circulation infarction (POCI) (Ramford et al. (1991), Lancet 337: 1521-1526). Subjects were classified according to smoking history as current smokers, former smokers, or non-smokers, and a medical history of previous stroke or transient ischemic attack (TIA), ischemic heart disease (IHD) and peripheral vascular disease (PVD) was documented. Atrial fibrillation (AF) was established on clinical examination at stroke presentation and confirmed by 12-lead electrocardiogram (Carter et al. (2007), see above). Presence of diabetes and hypertension was determined from the case notes and current use of hypoglycaemic and antihypertensive agents. Patients were flagged with the Office for National Statistics for notification of death, as previously described (Carter et al. (2007), see above). All subjects provided informed consent according to a protocol approved by the Leeds Teaching Hospitals Research Ethics Committee. Only patients who survived for longer than 30 days after the acute event with sufficient plasma available for analysis of SDMA were included in the present examination (n=394).

Venous blood samples were analysed for biochemical, haematological and haemostatic factors as previously described (Carter et al. (2007), Stroke, 38:1873-80). Estimated glomerular filtration rate (eGFR) was analysed according to the Cockroft-Gault equation ([140-age (years) x weight (kg)]/[0.814x creatinine [µmol/L)] x [0.85 for females]) adjusted for body surface area (BSA = 0.007184 x [weight (kg)]^{0.425} x [height (cm)]^{0.725}) expressed in ml/min/1.73 m² (Monami et al. (2009), Acta Diabetol, 46:191-6; Geddes et al. (2008), Nephrol Dial Tranplant, 23:4-6). Of the 394 individuals included in the present analysis, 95 did not have information on creatinine available and these subjects were excluded from analyses including eGFR.

Circulating levels of SDMA were determined by liquid chromatography-tandem mass spectrometry (LC-MS/MS) as described elsewhere (Schwedhelm et al. (2005), Clin Chem, 51:1268-71). A fully validated high throughput LC-MS/MS assay was used (Schwedhelm et al. (2007), J Chromatogr B Anal Technol Biomed Life Sci, 851:211-9). In brief, proteins were precipitated with methanol using 96-well 0.20-µm microfiltration plates pre-coated with 800 pmol of L-[²H₇]-arginine and 40 pmol of [²H₆]-ADMA (internal standards). After centrifugation, the microfiltrates were dried and analytes were converted to their butyl ester derivatives. Subsequent analyses were performed using a Chirobiotic T, 20 mm x 1.0 mm i.d. (inner diameter), microbore guard column (Astec, Whippany, NJ, USA) connected to a Varian 1200L Triple Quadrupols MS (Varian, Walnut Creak, CA, USA) in the positive electrospray ionisation (ESI+) mode. The sample run time was 1.6 min with an intra-assay precision of 3.5% (CV) for SDMA and an inter-assay precision of <5% (CV) for all variables.

The primary outcome measure was all-cause long-term mortality after acute ischemic stroke. Associations between variables were assessed by Spearman correlation coefficients (rho), wherein a rho>0.2 was regarded clinically relevant. Differences between two unrelated groups were compared using unpaired Student's t-tests and between >2 groups by one-way ANOVA. Differences between categorical data were analysed by χ2 test. Univariate associations between quartiles of SDMA and mortality were assessed using Kaplan-Meier survival analysis with significance determined using the log rank test. The association between SDMA and mortality was determined using multivariate Cox regression analyses including variables previously shown to be independently associated with mortality in this cohort (Carter et al. (2007), see above), with data presented as hazard ratios (with 95% confidence intervals). Statistical analyses were performed using SPSS for Windows v12.0 (SPSS Inc.).

Results: Cumulative survival decreased with ascending quartiles of SDMA. The hazard ratios (HR) for levels in the upper quartile compared with lower quartile were 3.70 (95% CI 2.46-5.57) for SDMA. SDMA was significantly correlated with a number of demographic, biochemical and haemostatic factors including age, albumin, creatinine, betaTG and vWF which were previously found to predict mortality in this cohort. SDMA was not significantly associated with previous history of stroke or stroke subtype. Most importantly, SDMA was significantly associated with and independently predictive of mortality. This suggests that SDMA is a prognostic marker for long-term all-cause mortality after ischemic stroke (see also Schulze et al. (2010), Artherosclerosis, 208: 518-23).

### EXAMPLE 2: Loci associated with SDMA plasma concentrations

An inverse-variance weighted meta-analysis was performed, using whole genome scans from the community-based Framingham Heart Study (FHS) cohort in order to identify genomic variations associated with plasma concentrations of SDMA.

### a) the samples used in the whole genome analysis

The Framingham Heart Study (Kannel, Feinleib et al. 1979) is a longitudinal observational, community-based cohort initiated in 1948 in Framingham, MA, to prospectively investigate risk factors for cardiovascular diseases. The Framingham Offspring Cohort consists of the children (and children's spouses) of the original Framingham cohort participants (Kannel, Feinleib et al. 1979). 2979 participants had complete genotypic information from the 550k gene array (as detailed below) and plasma levels for SDMA available.

The baseline characteristics of the FHS are shown in the below table.

**Table 2: Baseline characteristics of the FHS. Numbers are given as N, %, or mean; numbers in brackets indicate the standard deviation of the mean.**

| | **FHS** |
|---|---|
| **Clinical characteristics** | |
| Sample size | 2992 |
| Sex (male proportion) [%] | 46 |
| Age [Years] | 59.0 (9.7) |
| Systolic blood pressure [mm Hg] | 128 (19) |
| Diastolic blood pressure [mm Hg] | 75 (9) |
| BMI [kg/m²] | 27.89 (5.14) |
| Diabetes [%] | 11 |
| Current cigarette smoking [%] | 15 |
| | |

| **Biochemical characteristics** | |
|---|---|
| Serum creatinine [mg/dl] | 1.02 (0.18) |
| Plasma ADMA [µmol/l] | 0.55 (0.13) |
| Plasma L-arginine [µmol/l] | 78.9 (27.9) |
| Plasma SDMA [µmol/l] | 0.40 (0.10) |

### b) genotyping of the samples

For the Framingham Offspring cohort, the Human Mapping 500k Array Set and the 50K Humane Gene Focused Panel genotyping platform were used.

### c) determination of SDMA plasma concentrations

Plasma samples were stored frozen at -80°C without freeze thaw cycles until mass spectrometric determination was performed as described elsewhere (Schwedhelm et al. (2005), Clin Chem, 51:1268-1271) by using a fully validated high throughput LC-MS/MS assay (Schwedhelm et al. (2007), J Chromatogr B Analyt Technol Biomed Life Sci, 851:211-9). In brief, samples were processed by stable-isotope dilution and protein precipitation with methanol using 96-well 0.20-µm microfiltration plates (Millipore, Schwalbach, Germany). After centrifugation, the microfiltrates were dried and analytes were converted to their butyl ester derivatives. Subsequent analyses were performed using a Photobiotic T, 20 x 1.0 mm i.d., microbore guard column (Astec, Whippany, NJ, USA) connected to a Varian 1200L Triple Quadrupole MS (Varian, Walnut Creak, CA, USA) in the positive electrospray ionisation (ESI+) mode. The sample run time was 1.6 min with an intra-assay precision of 3.2% and an inter-assay precision of < 5% (CV).

### d) genome-wide association data analyses

SDMA plasma concentrations were analyzed for association using 2.2 million SNPs (imputed to the HapMAp CEU sample) as exposure variables, assuming an additive genetic model, and adjusting for sex, age, diabetes, systolic and diastolic blood pressure, smoking, BMI and serum creatinine. Additional adjustments were performed to account for the familial structure within the dataset using principal component analyses. Loci that reached genome-wide significance (defined as a p-value of 5x10⁻⁸) were considered genome-wide significant. A locus was defined as a set of SNP with pairwise correlation coefficients above 0.5 in the HapMap CAU sample.

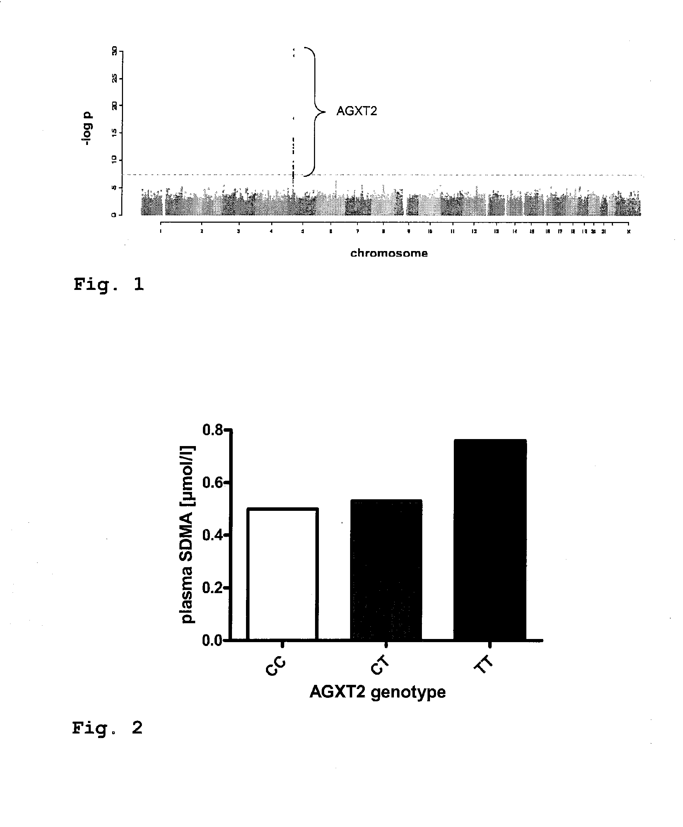

Results: SDMA plasma levels were associated with various SNPs marking the same locus 5p13 (Figure 1). The p-values for the hits show strong genome-wide significance for 11 SNPs: 2.87 x 10⁻³⁰- 3.54 x 10⁻⁹). This locus includes the AGXT2 gene (Figure 1).

**Table 3: Discovery association results for genetic loci associated with plasma levels of SDMA. The locus of all SNPs in the table is 5p13.**

| **SNP** | **SNP type** | **nearest gene** | **Major/ Minor Allele (MAF)** | **Effect size (SE)** | **p-value** |
|---|---|---|---|---|---|
| rs28305 (SEQ ID NO:1) | | | G/C | 0.031 | |
| | Intronic | AGXT2 | (0.095) | (0.003) | 2.87x10⁻³⁰ |
| rs37369 (SEQ ID NO:2) | | | C/T | 0.033 | |
| | CDS | AGXT2 | (0.080) | (0.003) | 2.21x10⁻²⁸ |
| rs1428281 | Intronic | AGXT2 | T/C | -0.017 | 1.23x10⁻¹⁵ |
| (SEQ ID NO:3) | | | (0.246) | (0.002) | |
| rs37370 (SEQ ID NO:4) | | | T/C | -0.032 | |
| | CDS | AGXT2 | (0.091) | (0.004) | 8.49x10⁻¹² |
| rs40200 (SEQ ID NO:5) | | | G/A | -0.031 | |
| | Intron | AGXT2 | (0.090) | (0.004) | 8.49x10⁻¹¹ |
| rs37379 (SEQ ID NO:6) | | | G/A | 0.031 | |
| | Intron | AGXT2 | (0.090) | (0.004) | 1.72x10⁻¹¹ |
| rs163594 (SEQ ID NO:7) | | | A/C | -0.014 | |
| | Intergenic | AGXT2 | (0.251) | (0.002) | 9.54x10⁻¹¹ |
| rs 10941225 (SEQ ID NO:8) | | | A/T | -0.010 | |
| | Intergenic | AGXT2 | (0.453) | (0.002) | 1.57x10⁻¹⁰ |
| rs153009 (SEQ ID NO:9) | | | A/G | 0.013 | |
| | Intergenic | AGXT2 | (0.242) | (0.002) | 4.08x10⁻¹⁰ |
| rs163585 (SEQ ID NO:10) | | | T/C | 0.012 | |
| | Intergenic | AGXT2 | (0.345) | (0.002) | 1.73x10⁻⁹ |
| rs163588 (SEQ ID NO:11) | | | A/G | 0.012 | |
| | Intergenic | AGXT2 | (0.336) | (0.002) | 3.54x10⁻⁹ |

The table 3 shows the primary findings from the genome-wide association analysis and the genome-wide p-values for the analyzed SNPs across the 22 autosomal chromosomes for SDMA plasma concentrations. One of the analyzed SNPs (rs37369) is located in the cod ing sequence of the AGXT2 and the affected amino acid is located in the catalytic active site of the AGXT2 enzyme. Patients carrying this mutation show higher SDMA plasma levels compared to patients carrying the wild type AGXT2 gene (Figure 2). This genetic association indicates that AGXT2 is an enzyme with catalytic activity to metabolize SDMA.

### EXAMPLE 3: Metabolism of SDMA to pyruvate by AGXT2 in mouse kidney tissue lysate

The assumed effect of AGXT2 on SDMA metabolism was confirmed by using mouse kidney tissue lysate with a total protein content of 250 µg as AGXT2 source. The mixture was incubated at 37°C for 60 minutes. Within this time a 96 well format plate was preloaded with 50µl of a solution containing 1mM NADH in 1mM Tris-HCl (pH 7.5). After incubation, 50µl of the reaction mixture were added to each well. AGXT2 activity was measured using a modified method described previously (Cooper et al. (2003), Biochem J, 376(Pt 1):169-78; Rodionov et al. (2010), J Biol Chem, 285(8): 5385-91). The reaction mixture contained (200µl) 6mM glyoxylate, 5mM dithiothreitol, 100 mM potassium phosphate buffer (pH 8), 60µM pyridoxal phosphate and 2µM of symmetric dimethylarginine (SDMA) as substrate.

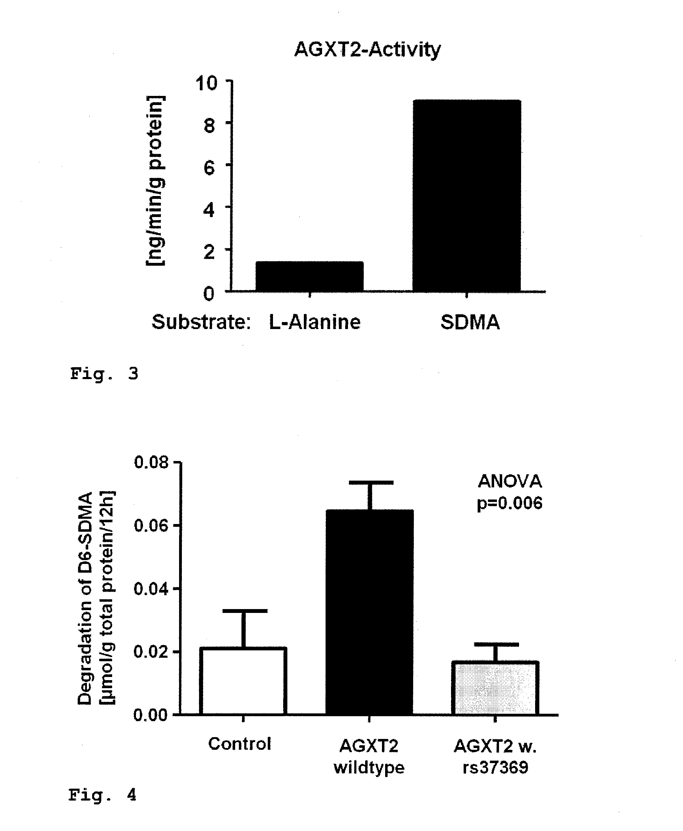

The transformation of SDMA by AGXT2 was determined indirectly by measuring the byproduct pyruvate. 10µl of lactate dehydrogenase (10 units) were added to the sample. With the help of the co-factor NADH pyruvate was converted to lactate. The change in NADH concentration was detected by measuring the absorbance at 340 nm. The decrease of NADH concentration correlates with the increase in pyruvate concentration and is a measure of AGXT2 activity (Figure 3).

Results: In the kidney homogenate incubated with the known substrate of AGXT2, L-alanine, as well as in the homogenate incubated with the novel substrate, SDMA, AGXT2 activity was detectable. Comparing both substrates, AGXT2 activity for SDMA is higher (Figure 3) than for L-alanine, suggesting a better binding profile for SDMA than for L-alanine to AGXT2.

### EXAMPLE 4: Association of AGXT2 genotypes with stroke outcome

Given the above results which show that SDMA is a predictor of total mortality after ischemic stroke, a potential association between AGXT2 genotypes and stroke as a clinical outcome was tested. For this purpose an association analysis was performed using a candidate gene approach in the stroke cohort of Leeds looking for different stroke subtypes and total mortality associated with AGXT2 genotypes.

The recruitment and characteristics of patients included in the LEEDS stroke study have been fully described elsewhere herein (compare Example 1).

The AGXT2 variants showing the strongest genome wide significance in the discovery meta-analysis and variants which are located in coding regions of the AGXT2 (rs37369, rs37370, rs28305, rs40200 (SEQ ID NOs:2, 4, 1, and 5, respectively) were used for analysis.

Results: Patients carrying the minor alleles of the four genotyped variants had overall significantly higher plasma SDMA levels. Univariate analysis revealed a trend across the different genetic groups toward a worse cumulative survival for the minor alleles. There were no significant differences in the AGXT2 genotype distributions of patients with haemorrhagic stroke, ischemic stroke and controls seen. However, looking at different subtypes of stroke the minor alleles of rs37370 and rs40200 (SEQ ID NOs:4 and 5) showed significant pairwise association with lacunar infarction (LACI) in comparison to total anterior circulation infarction (TACI).

### Example 5: Effect of certain AGXT2 alleles on AGXT2 enzyme activity

HEK cells which do not normally express AGXT2 were stably transfected to overexpress either the human AGXT2 wild-type gene (SEQ ID NO:13) or human AGXT2 containing the rs37369 (SEQ ID NO:2) variant, or empty control vector. The human cDNA of AGXT2 was purchased from Origene in a pCMV-XL5 backbone. The AGXT2 cDNA sequence was identical to GeneBank sequence NW_031900 (SEQ ID NO:13). The AGXT2 cDNA was inserted in the direct orientation into the XhoI and EcoRI site of the pcDNA3.1 vector. For transfection of human embryonic kidney (HEK) cells, FuGene reagent (Roche) was used. The transfection was performed according to manufacturer's protocol. To generate a stable AGXT2 overexpressing system the HEK-pcDNA3.1-AGXT2 cells were selected with 0.5 mg/ml G418.

To express the c.418C>T (rs37369) (SEQ ID NO:2) AGXT2 mutant, different AGXT2 cDNA fragments were generated by PCR using 5'-gcggaagttccatgactctaatctg-3' (SEQ ID NO:15) (underlined is an EcoRI restriction site), 5'-atggtgcttgtatgccacagg-3' (SEQ ID NO:16) (underlined is the mutation), 5'-cttcttccaccctccaatgcat-3' (SEQ ID NO:17) (with 5'-end phosphorylation) and 5'-gagctcgagtcgagttacttagc-3' (SEQ ID NO:18) (underlined is an XhoI restriction site). The activity of the AGXT2 mutant was determined relative to the non-mutated AGXT2.

AGXT2 activity was measured by incubating cells with 0.3 mmol/L pyridoxalphosphate, 1 mmol/L pyruvate and 1 mmol/L glyoxalate for 24 hours, then cells were washed in standard PBS (pH 7.4) buffer. Subsequently, the cells were resuspended and sonicated and incubated for 12h at 37°C in PBS buffer (pH 9.0) containing pyridoxalphosphate, 1 mmol/L puruvate and glyoxalate 1 mmol/L as well as 0.5 µmol/l deuterated SDMA (D6-SDMA). The concentration of D6-SDMA was quantified by using LC-MS/MS before and after incubation, and the difference in D6-SDMA concentration was expressed as µmol D6-SDMA/g of total protein/ 12 h.

Results: The degradation [µmol/g total protein/12 h] of D6-SDMA was significantly increased in HEK cells which were transfected with the human wildtype AGXT2 compared to those which were transfected with either the human AGXT2 containing the rs37369 (SEQ ID NO:2) variant or empty control vector (Figure 4).

## Claims

1. Method for determining/predicting the all-cause mortality risk of a test subject, comprising
(a) detecting in a sample from said test subject the expression level of the alanine-glyoxylate-aminotransferase 2 (AGXT2) gene;
(b) comparing the expression level of step (a) to the expression level of said gene in a reference subject;
wherein detection of an expression level of the AGXT2 gene which is significantly decreased in the test subject relative to the expression level in the reference subject indicates that the test subj ect has an increased mortality risk.

2. Method of claim 1, wherein the expression level of the AGXT2 gene in step (a) is detected by quantitative RT-PCR, TaqMan^{™} RT-PCR, real-time RT-PCR, or Northem-Blot analysis.

3. Method of any of claims 1-2, wherein the expression level of the AGXT2 gene in step (a) is decreased by at least 2-fold, at least 5-fold or at least 10-fold relative to the expression level in the reference subject.

4. Method for determining/predicting the all-cause mortality risk of a test subject, comprising
(a) detecting in a sample from said test subject the activity of the alanine-glyoxylate-aminotransferase 2 (AGXT2) enzyme;
(b) comparing said activity of step (a) to the activity of said enzyme in a reference subj ect;
wherein detection of an activity of the AGXT2 enzyme which is significantly decreased in the test subject relative to the activity in the reference subject indicates that the test subj ect has an increased mortality risk.

5. Method of claim 4, wherein the activity of the AGXT2 enzyme in step (a) is detected by ELISA, mass spectrometric methods or immunohistochemical methods.

6. Method of any of claims 4-5, wherein the activity of the AGXT2 enzyme in step (a) is decreased by at least 2-fold, at least 5-fold or at least 10-fold relative to the activity in the reference subj ect.

7. Method for determining/predicting the all-cause mortality risk of a test subject, comprising detecting in a biological sample a genetic alteration in the genome of said test subject, wherein said genetic alteration is associated with a decreased expression of the AGXT2 gene or with a decreased activity of the AGXT2 enzyme; wherein the detection of said genetic alteration indicates that the test subject has an increased mortality risk.

8. Method of claim 7, wherein said detection of a genetic alteration comprises the detection of at least one single nucleotide polymorphism (SNP).

9. Method of any of claims 7-8, wherein said single nucleotide polymorphism occurs in position 27 of any of the sequences depicted in SEQ ID NOs:1-11.

10. Method of any of the preceding claims, wherein the all-cause mortality is the all-cause mortality after having experienced a cardiovascular event, such as a stroke.

11. *In vitro* method for identifying a pharmaceutically active compound which decreases the all-cause mortality risk of a subject, comprising
(a) contacting a candidate compound with a cell that expresses the alanine-glyoxylate-aminotransferase 2 (AGXT2) gene;
(b) detecting whether said candidate compound alters the expression level of the AGXT2 gene;
wherein an increase of the expression level of the AGXT2 gene indicates a pharmaceutically active compound.

12. *In vitro* method for identifying a pharmaceutically active compound which decreases the all-cause mortality risk of a subject, comprising
(a) contacting a candidate compound with an alanine-glyoxylate-aminotransferase 2 (AGXT2) enzyme;
(b) detecting whether said candidate compound alters the activity of the AGXT2 enzyme;
wherein an increase of activity of the AGXT2 enzyme indicates a pharmaceutically active compound.

13. Compound which is effective in increasing the expression of the AGXT2 gene and/or the activity of AGXT2 enzyme for use in a method of decreasing the mortality risk of a subject.

14. Composition comprising AGXT2 enzyme for use in a method of decreasing the all-cause mortality risk of a subject.
